# EUROPEAN PATENT APPLICATION

(11) **EP 1 876 233 A1**
(43) Date of publication of application: **09.01.2008**
(21) Application number: 06712259.8
(22) Date of filing: 24.01.2006
(51) Int. Cl.: C12N 5/06, A61K 35/34, A61K 35/54, A61P 9/00, A61P 9/04, A61P 9/10, C07K 16/28, C12N 5/10, C12P 21/02, C12Q 1/02, C12N 15/09

(54) **CELLS CAPABLE OF DIFFERENTIATING INTO CARDIAC MUSCLE CELLS**

(30) Priority: 24.01.2005 JP 2005015539
(71) Applicant: Japan Health Sciences Foundation, Tokyo 103-0001 (JP)
(72) Inventor: UMEZAWA, Akihiro, nt, 2-10-1, Okura, Setagaya-ku Tokyo, 15 (JP); MIYOSHI, Shunichiro, Tokyo, 1510053 (JP); OGAWA, Satoshi, Tokyo, 1570066 (JP)
(74) Representative: Gillard, Richard Edward
(86) International application number: PCT/JP2006/301043
(87) International publication number: WO 2006/078034

(57) **Abstract**

Cells capable of differentiating into a cardiac muscle cell, which originate in an endometrial tissue or from an endometrial tissue isolated from a menstrual blood, or originate in mesenchymal stem cells isolated from a menstrual blood, a cord blood or an appendage of a fetus. The above-described cells can be relatively easily obtained from, for example, menstrual blood or cord blood, which can supply a large number of HLA tyes, or appendages of a fetus to be discharged after the birth. When cocultured with cardiac muscle cells, these cells differentiate into cardiac muscle cells at a high frequency. Moreover, the differentiation thereof into cardiac muscle cells arises at a high frequency without demethylating (for example, treating with 5-azacytidine) immortalized cells. By using the above-described cells, a drug for heart regeneration or a remedy for heart diseases can be provided.

## Description

### BACKGROUND OF THE INVENTION

### Field of Invention

This invention relates to cells capable of differentiating into cardiac muscle cells, which originate in a uterus membrane obtained from an endometrial tissue or a menstrual blood, or originates in mesenchymal stem cells obtained from a menstrual blood, a cord blood or an appendage of a fetus. More particularly, the present invention relates to a method for growing a cell capable of differentiating into a cardiac muscle cell, differentiation into a cardiac muscle cell, and a therapeutic agent for cardiac diseases using these cells.

### Background Art

Cardiac muscle cells actively conduct cell division in a perinatal period with autonomous beating. Simultaneously with birth, however, the cell division capability is lost, and, unlike hepatic cells, is not acquired again. Further, unlike skeletal muscle cells, the cardiac muscle cells do not possess undifferentiated precursor cells like satellite cells. Accordingly, when cardiac muscle cells undergo necrosis, for example, upon cardiac infarction, myocarditis or aging, cell hypertrophy rather than cell division of the remaining cardiac cells takes place in a body. Cardiac hypertrophy is physiologically adapted in an early stage. In concert with stromal fibrillation induced by the growth of coexisting fibroblasts, a deterioration in advanced function of the heart per se and, further, a deterioration in contractile function occur, resulting in the development of heart failure. Symptomatic treatment such as an enhancement in the force of cardiac contraction, a reduction in cardiac systolic overload and diastolic overload by vasodilators, or a reduction in blood flow by diuretics has hitherto been mainly adopted for the treatment of heart failure caused, for example, by cardiac infarction. On the other hand, cardiac transplantation is fundamental therapy for serious heart failure. Spreading of the cardiac transplantation to general medical treatment is not easy due to problems such as a lack of organ donors, difficulties involved in declaration of brain death, rejection, and medical cost inflation. In fact, cardiac diseases are the third most common cause of death in Japan (White Paper on Health and Welfare (1998)). The regeneration of lost cardiac muscle cells would lead to great advancement of medical welfare.

Up to now, AT-1 cells established from a tumor produced in the atrium of a transgenic mouse prepared by recombination of a promoter of an atrial natriuretic hormone with an SV40 large T antigen may be mentioned as a cell strain preserving the properties of cardiac muscle cells [Science, 239; 1029-1038 (1988)]. The cells, however, form a tumor upon in-vivo transplantation and thus are unsuitable for cell transplantation. Accordingly, in order to overcome the above problem, the following methods have been considered for regenerating cardiac muscles.

The first method is a method in which cells other than cardiac muscle cells are converted to cardiac muscle cells. This method has been analogized from the fact that the introduction of MyoD into fibroblasts can realize conversion to skeletal muscle cells. Up to now, a successful experience using P19 cells which are mouse fetal cancer cells has been demonstrated, but on the other hand, there is no report about any successful experience using noncancer cells [Cell Struc. & Func., 21: 101-110 (1996)].

The second method is a method for imparting again a cell division capability to cardiac muscle cells. This method is based on a phenomenon that the cardiac muscle can differentiate while beating in a fetal period. Up to now, however, any successful experience has not been reported.

The third method is a method in which cardiac cells are induced from undifferentiated stem cells. It has already been demonstrated that cardiac cells can be induced from embryonal stem cells (ES cells). The transplantation of embryonal stem cells per se to an adult poses problems such as the formation of carcinoma or antigenecity [Nature Biotechnology, 17, 139-142 (1999)].

Accordingly, in order to apply embryonal stem cells to actual medical treatment, a technique for purifying at least cardiac precursor cells or cardiac cells to a pure state is indispensable. It has been suggested that there is a possibility that the problem of antigenecity can be solved by a cloning technique. Since, however, a troublesome manipulation is necessary, the application of embryonal stem cells to general medical treatment is not easy.

A method in which cardiac precursor cells, which are undifferentiated cells, are acquired from an aborted fetus and are used for transplantation, has also been proposed. Regarding this method, it is known that, in an experiment using animals, the muscle precursor cells can effectively function as cardiac muscle cells [Science, 264, 98-101 (1994)]. In this method, however, the acquisition of a large amount of cardiac precursor cells is difficult, and, also from the viewpoint of ethics, the application of these cells to general medical treatment is not easy.

In addition to hematopoietic stem cells and vascular stem cells, mesenchymal stem cells are present in adult bone marrow. There is a report that bone cells, chondrocytes, tendon cells, ligamentous band cells, skeletal muscle cells, fat cells, stroma cells, and hepatic oval cells can be induced from the mesenchymal stem cells [Science, 284, 143-147 (1999); Science, 284, 1168-1170 (1999)]. On the other hand, it has recently been reported that cardiac muscle cells can be induced from cells acquired from the bone marrow of an adult mouse [J. Clinical Investigation, 103, 10-18 (1999)]. There is a report that up to about 50% of immortalized cells established from mouse bone marrow cells differentiate into myotube-like cells (US 2002/0142457). It has recently been reported that, also for human bone marrow cells, as with the mouse bone marrow cells, differentiation into cardiac muscle cells is possible [The Journal of Gene Medicine, 6, 833-845 (2004); Exp. Biol. Med., 229, 623-631 (2004)]. The differentiation efficiency, however, is lower than that in the mouse cells. Further, general anesthesia is necessary for harvesting bone marrow cells from the bone marrow, and the number of harvestable stem cells is small.

On the other hand, in the endometrium, the separation and regeneration of the tissue are periodically carried out, and the presence of tissue stem cells has been suggested. In fact, it has been found that a marker [CD117(c-kit), CD34] for certain stem cells is expressed in a human endometrium [Fertility and Sterility, 81, 403-407 (2004)], and an attempt to separate and identify tissue stem cells from the endometrium has also been reported [Australian and New Zealand Journal of Obstetrics and Gynaecology, 44, 380-386 (2004)]. Properties such as differentiating capability of tissue stem cells of which the presence in the endometrium is suggested, however, have not been fully elucidated yet, and there is no report about whether or not endometrium-derived tissue stem cells can differentiate into cardiac muscle cells. Further, any useful marker for separating and identifying stem cells capable of differentiating into cardiac muscle cells from the endometrium has not been reported.

An antibody, which can recognize various surface antigens, has been used for acquiring target cells from an in-vivo tissue. For example, it is known that immature hematopoietic stem cells have properties of CD34+/CD38-/HLA-DR-/CD90 (Thy-1)+ and that the development of CD38 and the disappearance of CD90 (Thy-1) occur as the hematopoietic stem cells differentiate [Tanpakushitsu Kakusan Koso (Protein, Nucleic Acid, and Enzyme,) Vol. 45, No. 13, 2056-2062 (2000)]. Markers, for example, CD34, CD31, Flk-1, Tie-2, and E-selectins are expressed in vascular endothelial cells [Bunshi Shinkekkanbyo (Molecular Cardiovascular Medicine) Vol. 1, No. 3, 294-302 (2000)]. On the other hand, markers such as CD90, CD105, and CD140a are expressed in mesenchymal stem cells of the bone marrow [Science, 284, 143-147 (1999); Science, 284, 1168-1170 (1999)]. Markers such as CD117 (c-kit) and CD140a are expressed in mouse bone marrow cell-derived cells capable of differentiating into cardiac muscle (US 2002/0142457), and markers such as CD13, CD29, CD44, CD55, CD59, CD90, CD105, and CD166 are expressed in human bone marrow derived cells capable of differentiating into cardiac muscle [The Journal of Gene Medicine, 6, 833-845 (2004)].

### SUMMARY OF THE INVENTION

The present inventors have now found that cells which originates in an endometrial tissue or from human endometrial tissue isolated from a menstrual blood, or mesenchymal stem cells obtained from a menstrual blood, a cord blood or an appendage of a fetus differentiate into cardiac muscle cells at a high frequency upon coculture with cardiac muscle cells. It has been found that differentiation into cardiac muscle cells occurs at a high frequency without demethylation (for example, 5-azacytidine treatment) of the cells. The present invention has been based on such finding.

Accordingly, an object of the present invention is to provide cells capable of differentiating into cardiac muscle cells.

Another object of the present invention is to provide a method for preparing cardiac muscle cells from cells capable of differentiating into cardiac muscle cells, which originate in an endometrium or from an endometrium isolated from a menstrual blood, or originates in mesenchymal stem cells isolated from a menstrual blood, a cord blood or an appendage of a fetus.

A further object of the present invention is to provide a medicament, especially a cardiac anagenetic agent or a therapeutic agent for cardiac diseases.

According to the present invention, there is provided a cell capable of differentiating into a cardiac muscle cell, which originates in an endometrial tissue or from an endometrial tissue isolated from a menstrual blood, or originates in mesenchymal stem cells isolated from a menstrual blood, a cord blood or an appendage of a fetus.

The cell according to the present invention can be obtained from a menstrual blood, a cord blood, or an appendage of a fetus to be discarded after delivery. Accordingly, in obtaining the cell, a relatively large amount of sample can be repeatedly harvested without newly inflicting pain. Further, the cell is very advantageous over conventional cells capable of differentiating into cardiac muscle cells, for example, in that a number of HLA types can be supplied.

The cell according to the present invention may have been immortalized. The immortalization may be carried out, for example, by intracellular expression of telomerase or Bmi-1, or intracellular expression of an E6 or E7 gene of a human papilloma virus.

According to the present invention, there is also provided a method for preparing a cardiac muscle cell, comprising at least: obtaining an endometrial cell from an endometrial tissue or a menstrual blood, or obtaining a mesenchymal stem cell isolated from a menstrual blood, a cord blood or an appendage of a fetus; and differentiation-inducing the cell into a cardiac muscle cell.

In a preferred embodiment of the present invention, in this method, the differentiation is carried out by at least one induction selected from the group consisting of coculture with a cardiac muscle cell, differentiation by demethylation of DNA, differentiation by a factor which develops in a fetal cardiogenesis region, or by a factor which acts on differentiation into a cardiac muscle cell at a fetal cardiogenesis stage, and differentiation by a culture supernatant of a cell capable of differentiating into a cardiac muscle cell, or a culture supernatant of a cardiac muscle cell differentiated from the cell.

In the method for preparing cardiac muscle cells according to the present invention, the endometrial cell may be immortalized before differentiation into a cardiac muscle cell. The immortalization may be carried out by intracellular expression of telomerase or Bmi-1, or intracellular expression of an E6 or E7 gene of a human papilloma virus.

Further, according to the present invention, there is provided a medicament comprising as an active ingredient the above cell. More specifically, the medicament is used as a cardiac anagenetic agent or a therapeutic agent for cardiac diseases.

Furthermore, according to another aspect of the present invention, there is provided a therapeutic agent for congenital heart diseases, comprising as an active ingredient the above cell into which a wild type gene relative to a mutant gene for congenital cardiac gene diseases has been introduced.

### BRIEF DESCRIPTION OF THE DRAWINGS

[Fig. 1] is a diagram showing the relationship between the proportion (percentage cardiac muscle induction) of differentiation of troponin I expressed cells from immortalized cells, and whether or not coculture is carried out and whether or not 5-azacytidine (5-azaC) treatment is carried out in Example 1 (3).
[Fig. 2] is a graph showing the results of the analysis of surface antigen of an E-MSC cell according to the present invention by flow cytometry using various antibodies.
[Fig. 3] is a graph showing the results of the analysis of surface antigen of an E-MSC cell according to the present invention by flow cytometry using various antibodies in the same manner as in Fig. 2.
[Fig. 4] is a graph showing the results of the analysis of surface antigen of an E-MSC cell according to the present invention by flow cytometry using various antibodies in the same manner as in Fig. 2.
[Fig. 5] is a graph showing the results of the analysis of surface antigen of an E-MSC cell according to the present invention by flow cytometry using various antibodies in the same manner as in Fig. 2.

### DETAILED DESCRIPTION OF THE INVENTION

### Deposition of cells

An E-MSC (hEPC100) cell and an E-MSC (hEPC214) cell, which are specific examples of cells capable of differentiating into cardiac muscle cells according to the present invention and will be described later, were deposited on January 19, 2005 (original deposition date) with International Patent Organism Depositary, National Institute of Advanced Industrial Science and Technology (Tsukuba Central 6, 1-1-1 Higashi, Tsukuba, Ibaraki, Japan) under accession numbers FERM BP-10467 and FERM BP-10468.

### 1. Isolation of cells capable of differentiating into cardiac muscle cells

The endometrium-derived cell capable of differentiating into a cardiac muscle cell according to the present invention can be isolated from an endometrium or a menstrual blood. A method for isolating the cell capable of differentiating into cardiac muscle cells will be described.
The method for obtaining an endometrial cell capable of differentiating into a cardiac muscle cell from a human endometrium is not particularly limited so far as the cell can be acquired in a safe and efficient manner. Specifically, the cell can be acquired by a method described in Am. J. Pathol., 163, 2259-2269 (2003).

Specifically, the human endometrial tissue harvested by surgery is cut finely, and the cell is placed in a culturable medium. The medium is not particularly limited so far as the cell can be cultured. A suitable example of the medium is a medium comprising α-MEM (α-minimum essential medium), DMEM (Dulbecco's modified Eagle's medium), IMDM (Isocove's modified Dulbecco's medium) or the like and 1 to 20% of an animal-derived serum, preferably 5 to 10% of a fetal calf serum (FCS), added to the medium. An antibiotic substance such as penicillin or streptomycin may be added. Further, in order to realize good cell separation, a method may also be adopted in which collagenase such as type 3 and DNA degradative enzyme such as deoxyribonuclease I are added to the medium and the mixture is gently shaken at 20 to 40°C, preferably 37°C, for example, for 10 min to 5 hr, preferably one hr. Each endometrial gland is separated with observation under a microscope and is cultured using a suitable culture vessel in an incubator, for example, a 24-well culture dish, under conditions of 37°C and 5% CO₂, whereby an endometrial cell capable of differentiating into a cardiac muscle cell can be acquired.

The mouse endometrial cell can be isolated by any method without particular limitation, for example, by a method described in McCormack SA and Glasser SR [Endocrinology, 106, 1634-1649 (1980)].

Specifically, uterus is sliced, and the slice is immersed in a protease, for example, PBS (phosphate-buffed saline) containing 5 mg/ml trypsin and 25 mg/ml pancreatin, and a reaction is allowed to react at 0 to 37°C for 10 min to 3 hr, preferably at 0°C for 30 min to 1 hr, and further at room temperature for 10 min to 3 hr, preferably for 30 min to 1 hr. After the enzyme-containing PBS solution is carefully removed, 1 to 20% of an animal-derived serum, preferably a suitable 5 to 10% FCS-containing medium, for example, a DMEM/F-12 (a mixture of DMEM and Ham's F-12 medium at a ratio of 1 : 1) medium, is added to deactivate trypsin. The medium is replaced with PBS, and the tissue is gently shaken for 10 sec to 30 min, preferably for 30 sec. The uterus tissue is carefully removed, and the cell suspension is passed through a filter, for example, a 70-µm mesh filter. The cell is recovered in a settled fraction, for example, by centrifuging the filtrate at 1000 x g for 5 min. The cell may be resuspended in PBS and centrifuged for washing. The cell settled by the centrifugation is suspended in a medium. This medium may be any one so far as the cell can be grown. For example, the medium may be a 1 to 20% animal-derived serum-containing, preferably 5 to 10% FCS-containing DMEM/F-12 medium. An antibiotic substance, for example, 100 units/ml penicillin and 100 mg/ml streptomycin, may be added to the medium. Further, a nonessential amino acid solution may also be added, for example, in an amount of 1%. Endometrium cells capable of differentiating into cardiac muscle cells may be acquired by using this method.

Further, the isolation of the cell from a menstrual blood may be carried out as follows. Since the number of cells acquired from the menstrual blood on the first day of menses is large, preferably the menstrual blood on the first day of menses (the first day on which the menses began) is harvested by a sanitary product or directly from the external genitalia. The harvested menstrual blood is placed preferably in a DMEM (Dulbecco's modified MEM) medium (penicillin-streptomycin added as antibiotic substance) containing a 1% fetal bovine serum, and the mixture is centrifuged to harvest cells contained in the menstrual blood. The cells are resuspended in a medium for cell culture, for example, a 10% fetal bovine serum(FBS)-containing α-MEM (α-modified MEM), DMEM (Dulbecco's modified MEM), or IMDM (Isocove's modified Dulbecco's medium) to give a menstrual blood-derived cell fluid.

Mesenchymal stem cells can be isolated from a menstrual blood, a cord blood, or an appendage of a fetus (for example, placenta, cord from the placenta, or amnion) as follows. Specifically, in DMEM, the harvested tissue is chopped (size: about 1 to 5 mm³) with an ophthalmic scissors, and the chopped tissue is allowed to stand for a few min. The supernatant containing blood cells is discarded, and the tissue is again washed with DMEM. This procedure is repeated three or four times. Thereafter, centrifugation may be carried out at 100 G for about 30 sec.

### 2 Culture of cells capable of differentiating into cardiac muscle cells

A conventional medium for cell culture (for example, Soshikibaiyo No Gijutukisohen (Technique for Tissue Culture, basic edition), Third Edition, Asakura Shoten 1996) may be used as a medium for culturing the cells, capable of differentiating cardiac muscle cells, isolated by the above method 1. Preferred media for cell culture are, for example, α-MEM, DMEM, DMEM/F-12 or IMDM, to which a bovine or other serum has been added in an amount of 5 to 20%. Conditions for culture are not particularly limited so far as cells can be cultured. The culture temperature is preferably 33 to 37°C. Further, preferably, the culture is carried out in an incubator filled with 5 to 10% of carbon dioxide gas. Preferably, endometrium-derived cells capable of differentiating into cardiac muscle cells are grown in such a state that is adhered onto a conventional plastic culture dish for tissue culture. The medium is removed around the time when the cells are grown on the whole area of a culture dish. A trypsin EDTA (ethylenediamine-N,N,N',N'-tetraacetic acid) solution is then added thereto to suspend the cells. The suspended cells are washed with PBS or the medium for cell culture and are diluted with the medium for cell culture by a factor of 5 to 20, followed by addition to a fresh culture dish to further conduct subculture.

### 3. Method for inducing cardiac muscle cells from cells capable of differentiating into cardiac muscle cells

Methods for differentiation of cells, capable of differentiating into cardiac muscle cells, into cardiac muscle cells include:
(1) coculture with cardiac muscle-derived cells,
(2) differentiation by treatment of DNA with a demethylating agent,
(3) differentiation by a factor expressed in a fetal cardiogenesis region, or a factor which acts on differentiation into cardiac muscle cells at a fetal cardiogenesis stage, and
(4) differentiation by a culture supernatant of cells capable of differentiating into cardiac muscle cells, or cardiac muscle cells differentiated from the cells.

In a preferred embodiment of the present invention, cardiac muscle cells can be induced in at a high percentage from cells capable of differentiating into cardiac muscle cells by using the method (1) alone or a combination of the method (1) with one or more methods selected from the methods (2) to (4). For example, the percentage induction is not less than 80% for the method (1) alone and is not less than 95% for a combination of the methods (1) and (2).

### (1) Coculture with cardiac muscle-derived cells

Cardiac muscle-derived cells are not particularly limited so far as the cells can be cocultured with cells capable of differentiating into cardiac muscle to promote differentiation into cardiac muscle cells. In a preferred embodiment of the present invention, the cardiac muscle-derived cell is a mammal-derived cell and is selected from the group consisting of, for example, mice, rats, guinea pigs, hamsters, rabbits, cats, dogs, sheep, pigs, cattle, goats, monkeys, and humans.

In a preferred embodiment of the present invention, mouse fetal cardiac muscle cells may be used as the cardiac muscle-derived cell. The mouse fetal cardiac muscle cell is not particularly limited and may be prepared by a method described, for example, in The Journal of Gene Medicine, 6, 833-845 (2004). In this method, the system and duration of pregnancy of pregnant mice are not particularly limited. A specific example of the pregnant mouse is a BALB/cAJcl mouse of the 14th to 16th day of pregnancy. A mother mouse is anaesthetized lightly with ether, and a lethal dose of pentobarbital is administered intraperitoneally for painless death. After the confirmation of the painless death, the mouse is subjected to laparotomy. In this case, preferably, the abdominal part is sterilized, for example, with Isodine for aseptic manipulation. The uterus is incised to extract a fetal mouse. The mouse is further sterilized with Isodine or 70% ethanol. The breast part is incised with ophthalmic scissors. The chest wall can be pressure-displaced to protrude the heart to the outside of the chest cavity.

The heart is excised with ophthalmic scissors and is suspended in a medium. In this case, the medium is not particularly limited. Preferred examples thereof include 5 to 10% bovine or other serum-containing DMEM media and DMEM/F-12 media. The number of fetal mice is not particularly limited. Preferably, however, hearts are extracted from 30 to 50 fetal mice and are suspended in the same medium. The suspended hearts are subjected to blunt cutting with ophthalmic scissors. A size small enough to allow a subsequent enzymatic reaction to proceed suffices for contemplated results. Preferably, however, chopping with scissors about 50 to 100 times is carried out until the original shape of the heart is lost. Cardiac muscle cells are isolated from the tissue using a protease. The protease is not particularly limited so far as it does not break the cardiac muscle and digests only tissue stroma. Specifically, the cardiac muscle tissue is introduced into PBS containing 0.05% trypsin and 0.25 mmol/L EDTA, and an enzymatic reaction is allowed to proceed at 20 to 40°C for 5 to 60 min, preferably at 37°C for 5 min. Thereafter, 1 to 20% of an animal-derived serum, preferably 5 to 10% of FCS-containing DMEM is used for stopping the enzymatic reaction. The tissue digestion fluid is centrifuged at 500 to 2000 rpm for 3 to 10 min. The centrifugation sediment is placed in a DMEM medium containing 5 to 10% of serum to suspend the cells. The cell suspension is cultured on a conventional dish for tissue culture for about 30 min to 3 hr, and only a supernatant containing cells having a low level of adherence is harvested to remove cells other than the cardiac muscle cells. The cells contained in the supernatant may be cultured under any conditions. Preferably, however, the cells are cultured at a cell density of 1 x 10³ to 1 x 10⁷/cm², preferably 5 x 10⁴ to 5 x 10⁵/cm², on a conventional tissue culture dish at a culture temperature of 33 to 37°C in an incubator filled with 5 to 10% of carbon dioxide gas.

Immediately after to 10 days after, preferably 1 to 2 days after, the initiation of culture of the primary cardiac muscle cells, cells capable of differentiating into cardiac muscle cells are added at a density of 1 x 10¹ to 1 x 10⁵/cm², preferably 5 x 10³/cm², onto culture cells. In order to confirm the differentiation of cells, capable of differentiating into cardiac muscle cells, into cardiac muscles, preferably, the cells capable of differentiating into cardiac muscle cells are labeled before the coculture. The label may be any one so far as the label is a cytotoxicity-free coloring matter which is not eluted in a culture solution during culture. Specifically, as described in The Journal of Gene Medicine, 6, 833-845 (2004), a method may be adopted in which a green fluorescent protein (GFP) gene is introduced with adenovirus into cells capable of differentiating into cardiac muscle cells, and the cells are observed as cells which emit green fluorescence.

After a few days, cells, which emit green fluorescence and move differently from primary mouse cultured cardiac muscle cells which contracts in a synchronized manner, can be observed, indicating that the cells capable of differentiating into cardiac muscle cells have differentiated into cardiac muscle cells. The action potential of the cardiac muscle may be measured, for example, using an inverted microscope and a fluorescent filter, whereby a cardiospecific potential waveform can be observed. Cardiac muscle makers can be detected by the expression of mRNA, for example, through RT-PCR, a Northern blotting analysis, or a DNA miroarray analysis, and the marker proteins can be detected, for example, by immunochemistry or Western blotting analysis, using antibodies specific for the proteins. Suitable marker genes/proteins include, for example, cardiac muscle transcription factors Nkx2.5/Csx, GATA4, TEF-1, MEF-2C, MEF-2D, MEF-2A, atrial natriuretic peptides (ANPs), brain natriuretic peptides (BNPs), α-myosin heavy chains (α-MHCs), β-myosin heavy chains (β-MHCs), myosin light chain-2a (MLC-2a), myosin light chain-2v (MLC-2v), α-cardiac actin, cardiac Troponin T, and connexin43 (Cnx43) [J. Clin. Invest., 103, 697-705 (1999)].

### (2) Differentiation by treatment of DNA with demethylating agent

The demethylating agent for DNA is not particularly limited so far as it is a compound which can induce demethylation of DNA, and examples thereof include demethylase, which is an enzyme capable of specifically inhibiting methylation of a cytosine residue in a CpG sequence in chromosome DNA, 5-azacytidine (hereinafter abbreviated to 5-aza-C), and DMSO (dimethyl sulfoxide). Demethylase described in Nature, 397, 579-583 (1999) may be mentioned as the demethylase. A specific example of differentiation by treatment of DNA with a demethylating agent will given below.
5-aza-C is added to the medium containing cells capable of differentiating into cardiac muscle cells to a concentration between 3 µmol/l and 10 µmol/l, and the mixture is incubated for 24 hr under the above culture conditions. The medium is replaced to remove 5-aza-C, and culture is further carried out for 2 to 3 weeks to acquire cardiac muscle cells. According to this method, preferably, for example, sinus node cells and ventricular cardiac muscle cells can be induced.

### (3) Differentiation by factor, which is expressed in fetal cardiogenesis region or factor which acts on differentiation into cardiac muscle cells at fetal cardiogenesis stage

Factors which are expressed in a fetal cardiogenesis region or factors which act on differentiation into cardiac muscle cells at a fetal cardiogenesis stage include cytokines, vitamins, adhesive molecules, and transcription factors.

The cytokine is not particularly limited so far as it can promote the differentiation of cells, capable of differentiating into cardiac muscle cells, into cardiac muscle cells at a cardiogenesis stage. Specific examples thereof include platelet-derived growth factors (hereinafter abbreviated to PDGFs), fibroblast growth factor 8 (FGF8), endothelin 1 (ET1), midkine, and bone morphogenetic factor 4 (BMP4). PDGFs include PDGF A, PDGF B, and PDGF C. The cytokine is used, for example, at a concentration of 10 to 40 ng/ml.
Further, the use of an inhibitor against cytokine which suppresses differentiation into cardiac muscle cells can promote the differentiation of cells capable of differentiating into cardiac muscle cells at a cardiogenesis stage. Cytokines which suppress differentiation into cardiac muscle cells include fibroblast growth factor-2 (hereinafter abbreviated to FGF-2), specifically FGF-2 represented by SEQ ID No. 7 or 8. Inhibitors against cytokines which suppress differentiation into cardiac muscle cells include substances which inhibit signalling of cytokine, for example, antibodies which neutralize the cytokine, and low-molecular compounds.

The vitamin is not particularly limited so far as it can promote the differentiation of cells capable of differentiating into cardiac muscle cells at a cardiogenesis stage. An example thereof is retinoic acid. Retinoic acid is used, for example, at a concentration of 10⁻⁹ mol/l.

The adhesive molecule is not particularly limited so far as it is expressed in a cardiogenesis region at a cardiogenesis stage. Specific examples of preferred adhesive molecules include extracellular matrix proteins, for example, gelatin, laminin, collagen, and fibronectin. For example, differentiation into cardiac muscle cells can be promoted by culturing cells capable of differentiating into cardiac muscle cells in a culture dish coated with fibronectin.

Transcription factors include homeobox-type transcription factor Nkx2.5/Csx, zinc finger-type transcription factor GATA4 belonging to a family of GATA, transcription factors MEF-2A, MEF-2B, MEF-2C, and MEF-2D belonging to a family of myocyte enhancer factor-2(MEF-2), dHAND, eHAND, and MesP1 belonging to a basic helix loop helix-type transcription factor, and TEF-1, TEF-3, and TEF-5 belonging to a family of TEA-DNA-binding-type transcription factor.

The above transcription factor can induce differentiation into cardiac muscle cells by introducing DNA coding for the factor into a cell capable of differentiating into cardiac muscle cells and expressing DNA.
Further, cells capable of differentiating into cardiac muscle cells can be induced into cardiac muscle cells by using a factor for inducing differentiation into cardiac muscle cells (hereinafter referred to as a cardiogenic factor). The cardiogenic factor can be acquired by adding various protease inhibitor to a culture supernatant of autonomous beating cells and subjecting the mixture to a combination of, for example, dialysis, salting out, and chromatography.

Further, a gene of a cardiac differentiation factor can be acquired by determining a partial amino acid sequence of the cardiogenic factor with a microsequencer and screening a cDNA library prepared from autonomous beating cells using a DNA probe designed based on the amino acid sequence.
The gene of the cardiogenic factor thus obtained is formulated into a myocardiogenic agent as follows.

(a) Myocardiogenic agent comprising as active ingredient gene coding for cardiac differentiation factor At the outset, a recombinant virus vector plasmid is constructed by inserting gene DNA fragment or full-length cDNA of a cardiac differentiation factor into the downstream of a promoter in a virus vector plasmid.
This recombinant virus vector plasmid is introduced into a packaging cell compatible with the virus vector plasmid.

The packaging cell is not particularly limited so far as the cell can replenish a deleted protein in a recombinant virus vector plasmid having a deletion of at least one gene coding for a protein necessary for virus packaging. For example, human kidney-derived HEK293 cells or mouse fibroblast NIH3T3 may be used.

The protein replenished by the packaging cell is a mouse retrovirus-derived gag, pol, env or other protein in the case of a retrovirus vector, an HIV virus-derived gag, pol, env, vpr, vpu, vif, tat, rev, nef or other protein in the case of a lentivirus vector, an adenovirus-derived E1A, E1B or other protein in the case of an adenovirus vector, and Rep (p5, p19, p40) and Vp(Cap) in the case of an adeno associated virus.

The virus vector plasmid used can produce a recombinant virus in the packaging cell and contains a promoter at a position where a wild type gene against a gene causative of congenital heart diseases can be transcripted in cardiac muscle cells.

Virus vector plasmids include MFG [Proc. Natl. Acad. Sci. USA, 92, 6733-6737 (1995)], pBabePuro [Nucleic Acids Research, 18, 3587-3596 (1990)], LL-CG, CL-CG, CS-CG, CLG [Journal of Virology, 72, 8150-8157 (1998)], and pAdex1 [Nucleic Acids Res., 23, 3816-3821 (1995)].

The promoter is not particularly limited for far as it can be expressed in a human tissue, and examples thereof include promoters of IE (immediate early) genes of cytomegalo virus (human CMV), early promoters of SV40, promoters of retrovirus, metallothionein promoters, heat shock protein promoters, and SRα promoters. Further, an enhancer of an IE gene of human CMV may be used together with the promoter. Further, a contemplated gene may can be expressed specifically in cardiac muscle cells by using a promoter of a gene specific for cardiac muscle cells, for example, the Nkx2.5/Csx gene.

A recombinant virus vector can be produced by introducing the recombinant virus vector plasmid into the packaging cell. The virus vector plasmid may be introduced into the packaging cell by a method, for example, a calcium phosphate method [Japanese Patent Laid-Open No. 227075/1990] or a lipofection method [Proc. Natl. Acad. Sci. USA, 84, 7413 (1987)].
Induction into cardiac muscle cells can be achieved by infecting the above recombinant virus vector with a cell capable of differentiating into cardiac muscle cells.

(b) Myocardiogenic agent comprising protein as active ingredient If necessary, based on full-length cDNA of a cardiogenic factor, a DNA fragment having a suitable length including a part coding for the protein is prepared.
A recombinant expression vector of the protein is constructed by inserting the DNA fragment or full-length cDNA into the downstream of a promoter within the expression vector.
The recombinant expression vector is introduced into a host cell compatible with the expression vector.

All of host cells which can express contemplated DNA can be used, and examples thereof include bacteria belonging to the genus Escherichia, the genus Serratia, the genus Corynebacterium, the genus Brevibacterium, the genus Pseudomonas, the genus Bacillus and the genus Microbacterium, yeasts belonging to the genus Kluyveromyces, the genus Saccharomyces, the genus Shizosaccharomyces, the genus Trichosporon and the genus Schwanniomyces, animal cells and insect cells.

The expression vector can be autonomously replicated or integrated into chromosome in the host cell and contains a promoter at a position where gene DNA of a cardiogenic factor can be transcripted.
When bacteria are used as the host cell, preferably, the recombinant expression vector of the cardiogenic factor can be autonomously replicated in the bacteria and, at the same time, comprises a promoter, a ribosome bonded sequence, DNA coding for a cardiogenic factor, and a transcription termination sequence. A gene for controlling the promoter may be contained.

Expression vectors include, for example, pBTrp2, pBTac1, and pBTac2 (all the above vectors being commercially available from Boehringer Mannheim), pKK233-2 (manufactured by Amersham Pharmacia Biotech), pSE280 (manufactured by Invitrogen), pGEMEX-1 (manufactured by Promega), pQE-8 (manufactured by QIAGEN), pKYP10 [Japanese Patent Laid-Open No. 110600/1983], pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene), pGEX (Amersham Pharmacia Biotech), pET-3 (manufactured by Novagen), pTerm2 (USP 4686191, USP 4939094, and USP 5160735), and pSupex, pUB110, pTP5, pC194, and pEG400 [J. Bacteriol., 172, 2392 (1990)].

The expression vector is preferably one in which the distance between a Shine-Dalgarno sequence as a ribosome bound sequence and an initiation codon has been regulated (for example, 6 to 18 bases).

The promoter is not particularly limited so far as it can be expressed in the host cell, and specific examples thereof include promoters derived from Escherichia coli or phages, such as trp promoter (Ptrp), lac promoter (Plac), P_{L}, promoter, P_{R} promoter, and T7 promoter, SPO1 promoter, SPO2 promoter, and penP promoter. Further, artificially designed and modified promoters such as promoter Ptrpx2 in which two Ptrp are provided in tandem, tac promoter, letI promoter [Gene, 44: 29 (1986)] and lacT7 promoter can also be used.

The yield of the contemplated protein can be improved by replacing a base in a base sequence in its part coding for a protein of gene DNA of a cardiogenic factor so that the codon is best suited for the expression of the host cell.
A transcription termination sequence is not always necessary for the expression of the gene of the cardiogenic factor. Preferably, however, a transcription termination sequence is provided just under a structural gene.

Host cells include cells of microorganisms belonging to the genus Escherichia, the genus Serratia, the genus Corynebacterium, the genus Brevibacterium, the genus Pseudomonas, the genus Bacillus and the genus Microbacterium, for example, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Bacillus subtilis, Bacillus amyloliquefaciens, Brevibacterium ammoniagenes, Brevibacterium immariophilum ATCC 14068, Brevibacterium saccharolyticum ATCC 14066, Corynebacterium glutamicum ATCC 13032, Corynebacterium glutamicum ATCC 14067, Corynebacterium glutamicum ATCC 13869, Corynebacterium acetoacidophilum ATCC 13870, Microbacterium ammmoniaphilum ATCC 15354 and Pseudomonas sp. D-0110.

The recombinant vector may be introduced by any method which can introduce DNA into the host cell. Examples of such methods include a method using a calcium ion [Proc. Natl. Acad. Sci. USA, 69, 2110 (1972)], a protoplast method (Japanese Patent Laid-Open No. 248394/1988), or a method described in Gene, 17, 107 (1982) and Molecular & General Genetics, 168, 111 (1979).
When the yeast is used as the host cell, for example, YEp13 (ATCC37115), YEp24 (ATCC37051), YCp50 (ATCC37419), pHS19, and pHS15 may be mentioned as the expression vector.

The promoter is not particularly limited so far as it can be expressed in the yeast, and examples thereof include PHO5 promoter, PGK promoter, GAP promoter, ADH promoter, gal1 promoter, gal10 promoter, heat shock protein promoter, MFα1 promoter, and CUP1 promoter.

Host cells include Saccharomyces cerevisiae, Schizosaccharomyces pombe, Kluyveromyces lactis, Trichosporon pullulans, and Schwanniomyces alluvius.

The recombinant vector may be introduced into DNA by any method without particular limitation so far as DNA can be introduced into the yeast, and examples thereof include an electroporation method [Methods in Enzymol., 194, 182 (1990)], a spheroplast method [Proc. Natl. Acad. Sci. USA, 75, 1929 (1978)], and a lithium acetate method [J. Bacteriol., 153, 163 (1983) and Proc. Natl. Acad. Sci. USA, 75, 1929 (1978)].

When the animal cell is used as the host cell, pCDNAI (manufactured by Invitorogen), pCDM8 (manufactured by Invitorogen), pAGE107 [Japanese Patent Laid-Open No. 22979/1991, and Cytotechnology, 3, 133 (1990)], pAS3-3 [Japanese Patent Laid-Open No. 227075/1990], pCDM8 [Nature, 329, 840 (1987)], pCDNAI/Amp (manufactured by Invitrogen), pREP4 (manufactured by Invitrogen), pAGE103 [J. Biochem. 101, 1307 (1987)], pAGE210 and the like may be exemplified as the expression vector.

The promoter is not particularly limited so far as it can be expressed in animal cells, and examples thereof include promoters of IE (immediate early) genes of cytomegalovirus (human CMV), early promoters of SV40, promoters of retrovirus, metallothionein promoters, heat shock protein promoters, and SRα promoters. Further, an enhancer of an IE gene of human CMV may be used together with the promoter.

Suitable host cells include human Namalwa cells, monkey COS cells, Chinese hamster CHO cells, and HBT5637 (Japanese Patent Laid-Open No. 299/1988).

The recombinant vector may be introduced by any method without particular limitation so far as DNA can be introduced into animal cells, and examples thereof include an electroporation method [Cytotechnology, 3, 133 (1990)], a calcium phosphate method (Japanese Patent Laid-Open No. 227075/1990), and a lipofection method (Proc. Natl. Acad. Sci., USA, 84, 7413 (1987), and Virology, 52, 456 (1973)). The acquisition and culture of a transformant may be carried out by a method described in Japanese Patent Publication H02(1990)-227075 A or Japanese Patent Publication H02(1990)- 257891 A.

When the insect cell is used as the host cell, the protein can be expressed by a method described, for example, in Baculovirus Expression Vectors, A Laboratory Manual, W. H. Freeman and Company, New York (1992), Current Protocols in Molecular Biology, Supplement 1-38 (1987-1997), Bio/Technology, 6, 47 (1988) or the like.
Specifically, a recombinant gene transfection vector and a baculovirus are cotransfected into an insect cell to obtain a recombinant virus in the culture supernatant of the insect cell, and then an insect cell is infected with the recombinant virus to express the protein.

Examples of gene transfection vectors suitable for use in this method are pVL1392, pVL1393 and pBlueBacIII (both products being manufactured by Invitrogen).

Examples of baculovirus include Autographa californica nuclear polyhedrosis virus infectable with an insect belonging to the family Barathra.

Examples of insect cells include Sf9 and sf21 [Baculovirus Expression Vectors, A Laboratory Manual, W. H. Freeman and Company, New York (1992)], which are ovary cells of Spodoptera frugiperda, and High 5 (manufactured by Invitrogen) which is an ovary cell of Trichoplusia ni.

Cotransfection of the recombinant gene transfection vector and the baculovirus into an insect cell for the preparation of a recombinant virus can be carried out, for example, by a calcium phosphate method [Japanese Patent Laid-Open No. 227075/1990] or a lipofection method [Proc. Natl. Acad. Sci. USA, 84, 7413 (1987)].

Regarding the expression of the gene, in addition to direct expression, for example, secretory production and fused protein expression may be carried out, for example, by a method described in Molecular Cloning, A Laboratory Manual, Second Edition, Cold Spring Harbor Laboratory Press (1989).
When the expression is carried out in yeasts, animal cells or insect cells, glycoproteins or glycosylated proteins can be obtained.

A cardiogenic factor can be produced by culturing a transformant carrying the recombinant DNA containing DNA coding for a cardiac muscle differentiation factor in a medium, producing and accumulating a cardiogenic factor in the culture, and harvesting the protein from the culture.
The transformant for the production of the protein as the cardiogenic factor can be cultured in the medium by any method commonly used in the culture of the host cell.

Regarding the medium for the culture of the transformant obtained using a procaryotic cell such as E. coli or a eucaryotic cell such as yeast as the host cell, any of natural media and synthetic media can be used so far as it contains a carbon source, a nitrogen source, an inorganic substance or the like which can be assimilated by the host cell and can efficiently culture the transformant.

The carbon source may be any one which can be assimilated by the host cell, and examples thereof include carbohydrates such as glucose, fructose, sucrose, molasses containing them, starch or starch hydrolyzate, organic acids such as acetic acid and propionic acid, and alcohols such as ethanol and propanol.

Carbon sources include ammonia, ammonium salts of various inorganic or organic acids such as ammonium chloride, ammonium sulfate, ammonium acetate and ammonium phosphate, and other nitrogen-containing compounds, and peptone, meat extract, yeast extract, corn steep liquor, casein hydrolyzate, soybean cake, soybean cake hydrolyzate, and various fermented cells and digested products thereof.

Examples of inorganic substances include primary potassium phosphate, secondary potassium phosphate, magnesium phosphate, magnesium sulfate, sodium chloride, ferrous sulfate, manganese sulfate, copper sulfate, and calcium carbonate.

The culture is carried out under aerobic conditions, for example, by shaking culture or submerged aeration spinner culture. The culture temperature is preferably 15 to 40°C, and the culture time is generally 16 hr to 7 days. The pH value is kept at 3.0 to 9.0 during the culture. The pH value is adjusted by the addition of an inorganic or organic acid, an alkaline solution, urea, calcium carbonate, ammonia or the like.
During the culture, if necessary, antibiotic substance such as ampicillin or tetracycline may be added to the medium.

When a microorganism transformed with an expression vector using an inducible promoter as a promoter is cultured, an inducer may be added to the medium. For example, when a microorganism transformed with an expression vector using lac promoter is cultured, for example, isopropyl-β-D-thiogalactopyranoside (IPTG) can be added to the medium. When a microorganism transformed with an expression vector using trp promoter is cultured, for example, indoleacrylic acid (IAA) can be added to the medium.

Media usable for the culture of a transformant obtained using an animal cell as a host cell include commonly used media such as RPMI1640 medium [The Journal of the American Medical Association, 199, 519 (1967)], Eagle's MEM medium [Science, 122, 501 (1952)], Dulbecco's modified MEM medium [Virology, 8, 396 (1959)] and 199 medium [Proceeding of the Society for the Biological Medicine, 73, 1 (1950)], or media prepared by adding an FCS or the like to these media.
The culture is generally carried out, for example, under conditions of pH 6 to 8, 30 to 40°C, and 5% CO₂ for 1 to 7 days.
During the culture, antibiotic substances such as kanamycin and penicillin may if necessary be added to the medium.

Media usable for the culture of a transformant obtained using an insect cell as the host cell include commonly used media such as TNM-FH media (manufactured by Pharmingen), Sf-900 II SFM media (manufactured by Life Technologies), ExCell400 and ExCell405 (all the above products being manufactured by JRH Biosciences), and Grace's Insect Medium [Grace, T. C. C., Nature, 195, 788 (1962)].
The culture is generally carried out, for example, under conditions of pH 6 to 7 and 25 to 30°C for 1 to 5 days.
Further, during the culture, an antibiotic substance such as gentamicin may, if necessary, be added to the medium.
A cardiogenic factor may be isolated and purified from the culture of the transformant by conventional protein isolation and purification methods.

For example, when the cardiogenic factor is expressed in a dissolved state in the cell, after the completion of the culture, the cell is collected by centrifugation, is suspended in an aqueous buffer solution, and is then disrupted with an ultrasonic disrupter, a French press, a Manton Gaulin homogenizer, a Dyno Mill or the like to give a cell-free extract. The cell-free extract is centrifuged, and a purified preparation can be produced from the resultant supernatant by a conventional protein isolation and purification method, that is, extraction with a solvent, salting-out, for example, with ammonium sulfate, desalting, precipitation with an organic solvent, anion exchange chromatography using resins such as diethylaminoethyl (DEAE)-Sepharose and DIAION HPA-75 (manufactured by Mitsubishi Chemical Corporation), cation exchange chromatography using resins such as S-Sepharose FF (manufactured by Amersham Pharmacia Biotech), hydrophobic chromatography using resins such as butyl Sepharose and phenyl Sepharose, gel filtration using a molecular sieve, affinity chromatography, chromatofocusing, and electrophoresis such as isoelectric focusing, either alone or in combination.

Further, when the protein is expressed as an insoluble substance in cells, the cells are collected, separated and disrupted, followed by centrifugation to collect the insoluble substance of the protein as a precipitate fraction.
The collected insoluble substance of the protein is solubilized with a protein-denaturing agent.

The solubilized solution is diluted or dialyzed to lower the concentration of the protein-denaturing agent in the solubilized solution and thus to return the structure of the protein to a normal three-dimensional structure, and the same isolation and purification method as described above is carried out to give a purified preparation of the protein.

When a cardiogenic factor or its derivative such as a glycosylated protein is extracellularly secreted, they can be recovered from the culture supernatant. That is, a culture supernatant is collected from the culture by centrifugation or the like, and a purified preparation can be obtained from the culture supernatant by the same isolation and purification method as described above.

The protein expressed by the above method may also be produced by a chemical synthetic method such as an Fmoc method (a fluorenylmethyloxycarbonyl method) or a tBoc method (a t-butyloxycarbonyl method). Alternatively, the synthesis may be carried out with a peptide synthesizer (for example, manufactured by Advanced ChemTech (USA), Perkin-Elmer, Amersham Pharmacia Biotech, Protein Technology Instrument (USA), Synthecell-Vega (USA), PerSeptive (USA), or Shimadzu Seisakusho Ltd.).

### (4) Differentiation by culture supernatant of cells capable of differentiating into cardiac muscle cells or cardiac muscle cells differentiated from the cells

Cells capable of differentiating into cardiac muscle cells can be induced into cardiac muscle cells by culture using a culture dish coated with an extracellular matrix acquired from autonomously beating cardiac muscle cells or by adding a culture supernatant of autonomously beating cardiac muscle cells.

The cell according to the present invention can be purified by any method without particular limitation, and examples of such methods include a panning method using an antibody which specifically recognizes cells capable of differentiating into cardiac muscle cells described later [J. Immunol., 141 (8), 2797-2800 (1988)], a FACS method [Int. Immunol., 10 (3), 275-283 (1998)], or a method in which a reporter system using a promoter of a gene specific for cells capable of differentiating into cardiac muscle cells is constructed.

### 4. Immortalization of cells capable of differentiating into cardiac muscle cells

Regarding myocardiogenic agents or therapeutic agents for heart diseases which will be described later, the necessity thereto should be separately considered. However, in an experiment for research for cells according to the present invention, since the number of times of division of endometrium-derived cells is usually finite, preferably, immortalization is carried out before the differentiation of cells capable of differentiating into cardiac muscle cells according to the present invention to increase the number of times of cell division.

The expression of telomerase or Bmi-1 in cells capable of differentiating into cardiac muscle cells may be mentioned as a method for immortalization which can increase the number of times of cell division without malignant transformation.

Methods for expressing cells capable of differentiating telomerase into cardiac muscle cells include a method in which the TERT gene as a catalyst subunit of telomerase is introduced into a retrovirus vector and the vector is introduced into cells capable of differentiating into cardiac muscle cells, a method in which a factor for induction expressing the TERT gene inherent in cells capable of differentiating into cardiac muscle cells is administered to cells capable of differentiating into cardiac muscle cells, and a method in which a vector containing DNA coding for a factor for induction expressing the TERT gene is introduced into cells capable of differentiating into cardiac muscle cells.

The factor for induction expressing the TERT gene can be screened by introducing a vector DNA, into which the TERT gene promoter and reporter genes such as GFP, luciferase or β-galactosidase have been integrated, into cells capable of differentiating into cardiac muscle cells.

Methods for expressing the Bmi-1 gene reported to be indispensable for self-renewal or self-replication of hematopoietic stem cells or neural stem cells, in cells capable of differentiating into cardiac muscle cells according to the present invention include a method in which the Bmi-1 gene is introduced into a retrovirus vector and the vector is introduced into cells capable of differentiating into cardiac muscle cells, a method in which a factor for induction expressing the Bmi-1 gene inherent in cells capable of differentiating into cardiac muscle cells is administered to cells capable of differentiating into cardiac muscle cells, and a method in which a vector containing DNA coding for a factor for induction expressing the Bmi-1 gene is introduced into cells capable of differentiating into cardiac muscle cells.

The factor for induction expressing the Bmi-1 gene can be screened by introducing a vector DNA, into which the Bmi-1 gene promoter and a reporter gene such as GFP, luciferase, or β-galactosidase has been integrated, into cells capable of differentiating into cardiac muscle cells.

The number of times of cell division can also be increased by a virus-derived immortalized gene. For example, the E6 gene and E7 gene of human Papillomavirus (HPV) may be used. Regarding the E6 gene, preferred are mutants of which the transforming activity to cells has been eliminated, for example, a mutant which lacks amino acid 151, i.e., leucine. Methods for expressing the gene in cells capable of differentiating into cardiac muscle cells according to the present invention include a method in which the gene is introduced into a retrovirus vector and this vector is introduced into cells capable of differentiating into cardiac muscle cells, a method in which the gene is introduced into a conventional plasmid vector and the vector is introduced into cells capable of differentiating into cardiac muscle cells, a method in which a structure capable of expressing the gene, for example, a structure comprising a promoter capable of expressing in an animal cell added to the upstream of the gene, a poly A signal added to the downstream of the gene and optionally an enhancer or a splicing signal for specific expression in desired cells added to the gene is introduced into cells capable of differentiating into cardiac muscle cells, and a method in which an HPV virus or the virus with deletion, substitution or addition of other sequence is infected with or introduced into cells capable of differentiating into cardiac muscle cells.

### 5. Myocardiogenic agent or therapeutic agent for heart diseases, comprising cell capable of differentiating into cardiac muscle cell

The cell according to the present invention can differentiate into a cardiac muscle cell. According to another aspect of the present invention, there is provided a medicament comprising the cell according to the present invention, specifically a myocardiogenic agent or a therapeutic agent for heart diseases.

Heart diseases which should be treated by the myocardiogenic agent or therapeutic agent for heart diseases according to the present invention include cardiac infarction, ischemic heart diseases, congestive heart failure, arrhythmia, hypertrophic cardiomyopathy, dilated cardiomyopathy, myocarditis, and valvular diseases.

Cells contained in the medicament according to the present invention may if necessary have been immortalized, or alternatively may be cells which have been induced into cardiac muscle cells or cardiac muscle precursor cells. In a preferred embodiment of the present invention, cells which can be induced into various cardiogenic cells, for example, endocardial endothelial cells, cushion cells, ventricle-type cardiac muscle cells, atrium-type cardiac muscle cells, and sinus node cells, or induced cells thereof or their precursor cells are used according to affected sites of the heart.

For example, a method utilizing a catheter is used for transporting a medicament to an affected site. A specific method will be described by taking an ischemic heart disease as an example. Since the cardiac muscle cell affected by an ischemic heart disease is present on the downstream of a vasoconstricted site, before the injection of the above cell, the vasoconstricted site should be identified by coronary angiography (Zusetsu Byotai Naikakoza Junkanki-1 (Illustrative Pathologic Medical Course; Cardiovascular System-1), MEDICAL VIEW, 1993). Organic stenosis lesions are classified into afferent stenosis, eccentric stenosis, and multiple wall irregularity according to stenotic conditions. In particular, eccentric stenosis is subclassified into two types, type I and type II. The form of stenosis is known to be related to the progress and prognosis of stenocardia. Type II eccentric stenosis and multiple wall irregularity are mainly found in unstable stenocardia and are highly likely to be turned into cardiac infarction. When the blood vessel has become completely stenosed, there is a possibility that the cell introduced does not arrive at the affected site. Accordingly, the stenosed site should be previously reopened, for example, by percutaneous transluminal coronary angioplasty (PTCA) or thrombolytic therapy. Cells introduced may be classified into ventricle type and atrium type according to the site of the affected cardiac muscle cell. The catheter can be inserted by a Sones method in which the catheter is introduced through upper right brachial artery (Zusetsu Byotai Naikakoza Junkanki-1 (Illustrative Pathologic Medical Course; Cardiovascular System-1), MEDICAL VIEW, 1993) or a Jundkins method in which a catheter is inserted through femoral artery (Zusetsu Byotai Naikakoza Junkanki-1 (Illustrative Pathologic Medical Course; Cardiovascular System-1), MEDICAL VIEW, 1993).

### 6. Utilization in treatment of congenital gene diseases

In another embodiment of the present invention, there are provided a treating method or therapeutic agent for a cardiac-related congenital gene disease.
Among diseases causative of heart failure, a group of diseases cause heat failure due to deletion of a part of protein necessary for cardiac differentiation or maintenance due to mutation of a single gene. Such diseases include, for example, familial hypertrophic cardiomyopathy, Fabry's diseases, long QT syndromes, Marfan's syndromes, aortic stenosis, mitochondrial cardiomyopathy, and Duchenne muscular dystrophy. These diseases are known to be caused by gene abnormality of myosin, troponin, tropomyosin, voltage-dependent Na channel, K channel, fibrin, elastin, mitochondria, and dystrophin [Chiryogaku (Biomedicine and Therapeutics), 30, 1302-1306 (1996)].

According to the present invention, there is provided a method for treating the above diseases. This method comprises acquiring, from a patient, a cell capable of differentiating into a cardiac muscle cell, introducing a normal gene into the cell, and then transplanting this cell to the heart, for example, in the same manner as in the medicament according to the present invention. Here a method may be adopted in which the normal gene is inserted into a vector for gene therapy described in the above item 3 (3) (b) and is then introduced into a cell capable of differentiating into a cardiac muscle cell according to the present invention.

### 7. Acquisition of antibody capable of specifically recognizing surface antigen specific for cell capable of differentiating into cardiac muscle cell

A method for preparing an antibody capable of specifically recognizing a surface antigen expressed in a cell capable of differentiating into a cardiac muscle cell according to the present invention will be described.

The antibody capable of recognizing a surface antigen expressed specifically in a cell capable of differentiating into a cardiac muscle cell according to the present invention can be used in purity test and purification of a cell capable of differentiating into a cardiac muscle cell necessary for carrying out cellular therapy of heart diseases such as cardiac infarction.

This antibody can be acquired by administering as an antigen cells capable of differentiating into cardiac muscle cells (3 to 5 × 10⁵ cells/animal) according to the present invention or a cell membrane fraction prepared from the cells (1 to 10 mg/animal), together with a suitable an adjuvant, for example, a complete Freund's adjuvant, an aluminum hydroxide gel, a vaccine against Bordetella pertussis, subcutaneously, intravenously or intraperitoneally to a nonhuman mammal such as a rabbit, a goat or three- to twenty-week old rat, mouse, or hamster.

After the first administration, the antigen is administered every one to two weeks three to 10 times. On the third to seventh day after each administration, blood is collected from a venous plexus at the back of the eye, and whether or not the serum is reacted with the antigen used for immunization, for example, by enzyme-linked immunosorbent assay [Koso Meneki Sokuteiho (Enzyme-Linked Immunosorbent Assay) ELISA: Igakushoin 1976, and Antibodies-A Laboratory Manual, Cold Spring Harbor Laboratory, 1988].
A nonhuman mammal of which the serum has a satisfactory antibody titer against the antigen used for immunization is used as a supply source for serum or antibody producing cells.
A polyclonal antibody may be prepared by isolating and purifying the serum.
A monoclonal antibody may be prepared by hybridizing the antibody producing cell with a nonhuman mammal-derived myeloma cell to prepare a hybridoma, culturing the hybridoma or administering the hybridoma to an animal for ascites canceration, and isolating and purifying the culture or ascites.

Antibody producing cells include splenocytes, lymph nodes, and antibody producing cells in peripheral blood. Splenocytes are particularly suitable.

Suitable myeloma cells include mouse-derived established cell lines, for example, 8-azaguanine-resistant mouse (BALB/c-derived) myeloma cell lines, that is, P3-X63Ag8-U1 (P3-U1) cell line [Current Topics in Microbiology and Immunology, 18, 1 (1978)], P3-NS1/1-Ag41 (NS-1) cell line [European J. Immunology, 6, 511 (1976)], SP2/O-Ag14 (SP-2) cell line [Nature, 276, 269 (1978)], P3-X63-Ag8653 (653) cell line [J. Immunology, 123, 1548 (1979)], and P3-X63-Ag8 (X63) cell line [Nature, 256, 495 (1975)].
Hybridoma cells can be prepared in the following manner.

Antibody producing cells and myeloma cells are mixed together, and the mixture is suspended in an HAT medium [a medium prepared by adding glutamine (1.5 mmol/l), 2-mercapto ethanol (5 × 10⁻⁵ mol/l), gentamycin (10 µg/ml), and 10% FCS to an RPMI-1640 medium, and further adding hypoxanthine (10⁻⁴ mol/l), thymidine (1.5 × 10⁻⁵ mol/l), and aminopterin (4 × 10⁻⁷ mol/l)] and is then cultured for 7 to 14 days. After the culturing, a part of the culture supernatant is sampled and tested, for example, by enzyme immunoassay to select those which can react with the antigen but not with protein which does not contain the antigen. Subsequently, cloning is carried out by a limiting dilution analysis, and a hybridoma which shows stable and high antibody titer by enzyme immunoassay is selected as monoclonal antibody producing hybridoma cells.

For the isolation and purification of the polyclonal antibody or monoclonal antibody, centrifugation, ammonium sulfate precipitation, caprylic acid precipitation, or chromatography using a DEAE-Sepharose column, an anion exchange column, a protein A or G column, a gel filtration column and the like may be employed. They may be used either alone or in a combination of two or more.

Whether or not the sample cell expresses the specific surface antigen can easily be examined by providing the antibody, acquired by the above method, capable of specifically recognizing the surface antigen expressed in cells capable of differentiating into cardiac muscle cells and comparing the reactivity with the sample cell and the reactivity with control cells such as hematopoietic stem cells or nervous stem cells.

### 8. Acquisition of surface antigen expressed in cell capable of differentiating into cardiac muscle cell, and gene coding for the surface antigen

A subtraction method [Proc. Natl. Acad. Sci. USA 85, 5738-5742 (1988)] and a representational difference analysis [Nucleic Acids Research, 22, 5640-5648 (1994)], in which genes, which take different expression form between samples different from each other in origin, are acquired, may be mentioned as a method for acquiring a surface antigen gene expressed specifically in a cell capable of differentiating into a cardiac muscle cell according to the present invention.

At the outset, a cDNA library prepared from the cell capable of differentiating into a cardiac muscle cell is subjected to subtraction using mRNA acquired from a control cell other than the cell capable of differentiating into a cardiac muscle cell, for example, a hematopoietic stem cell or a nervous stem cell. A subtracted cDNA library with a high content of a gene specifically expressed in the cell capable of differentiating into a cardiac muscle cell is prepared. Thereafter, a base sequence analysis of inserted cDNA in the subtracted cDNA library is carried out from the 5' terminal side randomly to select only those having the secretion signal sequence (random sequence analysis). Whether the protein encoded by the cDNA is a secretory protein or a membrane protein can be determined by determining the full length base sequence of the cDNA thus obtained.

In the above process, a signal sequence trap method can be used instead of the random sequence analysis (Science, 261: 600-603 (1993) and Nature Biotechnology, 17: 487-490 (1999)). The signal sequence trap method is a method for selectively screening genes having a secretion signal sequence.

In order to efficiently acquire the specific surface antigen, a method is preferably adopted in which a signal sequence trap library is prepared using a vector suitable for subtraction, and the signal sequence trap library prepared from the cell capable of differentiating into a cardiac muscle cell is subjected to subtraction using mRNA obtained from control cells such as hematopoietic stem cells or neural stem cells. The DNA fragment containing a secretion signal sequence thus obtained can be used as a probe for cloning the full length cDNA.
Whether the protein encoded by the cDNA is a secretory protein or a membrane protein can be determined by analyzing the full length base sequence of the full length cDNA.

Even in the use of the random sequence analysis or signal sequence trap method, when the obtained clone codes for a membrane protein, the specific antibody can be acquired by the above method by preparing a synthetic peptide based on an amino acid sequence analogized from the base sequence and using the synthetic peptide as an antigen.

Some of membrane proteins code for a receptor. This receptor possibly acts on specific growth of cells capable of differentiating into cardiac muscle cells, or the modulation of differentiation into cardiac muscle cells, and can be used in search for a ligand of the receptor. In the case of the secretory protein, the secretory protein can be used directly for the growth or differentiation of cells capable of differentiating into cardiac muscle cells.

### 9. Methods for screening for growth factor for cells capable of differentiating into cardiac muscle cells and factor for differentiation into cardiac muscle cells

Screening a growth factor for cells capable of differentiating into cardiac muscle cells and a factor for their differentiation into cardiac muscle cells can be carried out by culturing the cells capable of differentiating into cardiac muscle cells in a serum-free medium in the presence of various test substances and examining whether the cells are grown or induced into cardiac muscle cells.

Test substances include, but are not limited to, secretory proteins such as various cytokines and growth factors, membrane-bound proteins such as cell adhesion molecules, tissue extracts, synthetic peptides, synthetic compounds, and culture broths of microorganisms.
The growth ability can be evaluated by examining the colony forming ability, the BrdU uptake and the like.
The colony forming ability can be determined by scattering the cell capable of differentiating into a cardiac muscle cell according to the present invention at a low density.
The BrdU uptake can be examined by immunostaining using an antibody capable of specifically recognizing BrdU.

Methods for evaluating differentiation into cardiac muscle cell include a method using spontaneous beating of the cell as an indicator and a method using the expression of a reporter gene introduced into the cell as an indicator.

The method using the expression of a reporter gene introduced into the cell as an indicator is a method in which a vector DNA with a promoter of a gene expressed specifically in a cardiac muscle cell and a reporter gene integrated thereinto is introduced into a cell capable of differentiating into a cardiac muscle cell and the expression of the reporter gene is examined using the cell.

Reporter genes include genes coding for GFP, luciferase or β-galactosidase.

Promoters of a gene expressed specifically in a cardiac muscle cell include cardiac Troponin I [J. Biological Chemistry, 273, 25371-25380 (1998)].

### 10. Method for isolating cell, capable of differentiating into cardiac muscle cell, using antibody

According to another aspect of the present invention, there is provided a method for acquiring a cell, which expresses a target surface antigen, from an endometrium or a menstrual blood. Method usable herein include a method using a flow cytometer having a sorting function and a method using magnetic beads.

Sorting methods for the flow cytometer include water droplet charging and cell capture (Furo Saito Meta Jiyu Jizai (Perfect Manipulation of Flow cytometer), p14-23, Shujunsha, 1999). In both the methods, the expression level of the antigen can be quantitatively determined by converting fluorescence intensity, emitted from the antibody bonded to molecules expressed on the surface of the cell, to an electric signal. Further, a combination of types of fluorescence used can realize isolation utilizing a plurality of surface antigens. Fluoroescences include FITC (fluorescein isothiocyanate), PE (phycoerythrin), APC (Allo-phycocyanin), TR (TexasRed), Cy3, CyChrome, Red613, Red670, PerCP, TRI-Color, and QuantumRed (Furo Saito Meta Jiyu Jizai (Perfect Manipulation of Flow Cytometer), p3-13, Shujunsha, 1999).

Staining methods include a method in which cells are isolated from an endometrium or a menstrual blood by the above method followed by staining directly with an antibody, and a method in which culture and growth are once carried out in a suitable medium followed by staining with an antibody.

For staining of the cell, at the outset, a primary antibody capable of recognizing a surface antigen is mixed with a contemplated cell sample, and the mixture is incubated on ice for 30 min to 1 hr. When the primary antibody has been labeled with fluorescence, washing is followed by isolation with a flow cytometer. When the primary antibody has not been labeled with fluorescence, a method is adopted in which, after washing, a secondary antibody, labeled with fluorescence, having bonding activity to the primary antibody is mixed with the cell reacted with the primary antibody and the mixture is again incubated on ice for 30 min to 1 hr. After washing, the cell stained with the primary antibody and secondary antibody is isolated with a flow cytometer.

In the method using magnetic beads, a large amount of cells which express a target surface antigen can be isolated. The isolation purity in this method is inferior to that in the method using the flow cytometer. However, a satisfactorily high cell purity can be ensured by repeating the purification procedure.

After the reaction of the cell with a primary antibody, the primary antibody in its part remaining unreacted with the cell is removed, and a secondary antibody, which can be specifically bonded to the primary antibody and to which magnetic beads have been bonded, is then bonded to the cell. The cell from which the residual secondary antibody has been removed by washing can be isolated using a stand provided with a magnet. Materials and apparatus necessary for these procedures are available from Dynal.

The method using magnetic beads can also be used in removing unnecessary cells from a cell sample. A StemSep method commercially available from Stem Cell Technologies Inc. (Vancouver, Canada) can be used to more efficiently remove unnecessary cells.

Antibodies usable in the above methods include the antibodies acquired in the above 8, antibodies which recognize Flk-1 and CD31 known as a surface antigen of vascular endothelial cells, antibodies which recognize CD13, CD14, CD34, CD45, and CD90 known as a surface antigen of hematopoietic cells, antibodies which recognize CD140a (PDGFRα) known as a surface antigen of mesenchymal cells, antibodies which recognize integrin CD29, antibodies which recognize matrix receptors CD44, CD50, and CD54, antibodies which recognize CD24 as a marker of B cells or granulocytes, CD55 and CD59 widely expressed in blood cells, and antibodies which recognize adhesive molecule CD166 as a member of an immunoglobulin superfamily. When these antibodies are used in combination, higher-purity target cells can be obtained.

Specifically, CD34-negative, CD29-positive, and CD140a-negative cells and CD44-positive cells can be acquired by removing CD34-positive cells and CD140a-positive cells from cells derived from a human endometrium or a menstrual blood, for example, using the above method utilizing immunomagnetic beads, and then fractionating CD29-positive and CD44-positive cell fractions to isolate a target cell.

### 11. Isolation of cardiac muscle precursor cells using cardiac muscle specific gene promoter reporter vector

In order to efficiently isolate cardiac muscle cells or cardiac muscle cell precursor derived from cells capable of differentiating into cardiac muscle cells, a green fluorescent protein (GFP) of luminous Aequorea can be used as an indicator for cell isolation.

Specifically, the GFP gene is ligated to the downstream of a promoter of a gene specifically expressed in cardiac muscle cells, or a gene specifically expressed in cells capable of differentiating into cardiac muscle cells acquired in the above item 9 to prepare a vector which is then introduced into a cell capable of differentiating into a cardiac muscle cell. The cell into which the reporter vector has been introduced is isolated using, for example, drug resistance as an indicator and is then induced into cardiac muscle cells. Induced cells express GFP and emit fluorescence. Cardiac muscle cells and cardiac muscle precursor cells which have emitted fluorescence can easily be isolated with a flow cytometer (Furo Saito Meta Jiyu Jizai (Perfect Manipulation of Flow cytometer), p44-52, Shujunsha, 1999).

MLC2v and Troponin I may be used as the promoter of a gene specifically expressed in cardiac muscle cells.

For example, the above-described plasmid vectors and adenovirus vectors for animal cells can be used as the vector.

### EXAMPLES

The present invention is further illustrated by the following Examples that are not intended as a limitation of the invention.

### Example 1

Acquisition of endometrium-derived cells capable of differentiating into cardiac muscle cells from human menstrual blood, and culture of the acquired endometrium-derived cells

### (1) Isolation of cells from human menstrual blood and culture of the cells

A menstrual blood on the first day of a menstrual period (the day of the start of a menstrual period) was transferred through a sanitary product or directly from an genital area to a 10-ml sterile plastic dish. The menstrual blood harvested in the plastic dish was moved to a 50-ml plastic tube containing 20 ml of a culture medium [1% fetal bovine serum-containing DMEM (Dulbecco's modified MEM) medium; penicillin/streptomycin antibiotic added]. The culture containing the acquired menstrual blood was centrifuged at 1,000 × g to collect cells contained in the menstrual blood. The menstrual blood cells were resuspended in an α-MEM (α-modified MEM) medium containing 10% FBS (fetal bovine serum) to a menstrual blood-derived cell fluid.

The Isolated cells contained in the menstrual blood were dispersed in an α-MEM medium containing 10 to 20% of a bovine serum or the like. The dispersion was placed in a plastic culture dish for tissue culture and was cultured in an incubator filled with 5 to 10% carbon dioxide gas at a culture temperature of 33 to 37°C. On the second day from the start of culture, medium replacement was carried out for the removal of erythrocytes. The medium was removed around the time when the cells were grown over the whole area of the culture dish. A trypsin EDTA solution was added to suspend the cells.

### (2) Gene introduction into human endometrium-derived cells, and establishment of immortalized cells

An immortalizing gene was introduced into the human endometrium-derived cell obtained in the above (1) using retrovirus vector to establish a human endometrium-derived immortalized cell line.
That is, on the day before virus infection, 5 × 10⁶ cells were inoculated on a 6-cm culture dish using a DMEM (Dulbecco's modified MEM) medium containing a 10% fetal calf serum (FCS) and were cultured in an incubator under conditions of 37°C and CO₂ concentration 5% overnight. Hexadimethrine bromide (polybrene) (manufactured by Sigma) was added to a final concentration of 8 µg/ml to a solution containing a human papilloma virus (HPV)16-derived retrovirus vector LXSN-16E7, which expresses E7, prepared by the method described in a document [J. Gene Med., 6, 833-845 (2004)]. The supernatant (2 ml) of the culture of the human endometrium-derived cells was replaced with 2 ml of the virus liquid, followed by culture in an incubator under conditions of 37°C and CO₂ concentration 5% overnight. The medium was replaced with the same medium containing 100 µg/ml G418, and the culture was continued under the same conditions to obtain a cell line in which a retrovirus vector LXSN-16E7 has been incorporated.

The cell line thus obtained was further infected with a human TERT (hTERT)-containing retrovirus vector LXSH-hTERT Specifically, the cell line was grown in the same medium containing 100 µg/ml G418, and, on the day before infection of 5 × 10⁶ cells with virus, was inoculated on a 6-cm culture dish and was cultured in an incubator under conditions of 37°C and CO₂ concentration 5% overnight. Polybrene was added to a final concentration of 8 µg/ml to a solution containing a retrovirus vector LXSH-hTERT, which expresses hTERT, prepared by the method described in a document [J. Gene Med., 6, 833-845 (2004)]. The supernatant (2 ml) of the culture of the human endometrium-derived cell line in which an HPV16-derived E7 gene had been incorporated, was replaced with 2 ml of the virus liquid, followed by culture in an incubator under conditions of 37°C and CO₂ concentration 5% overnight. The medium was replaced with the same medium containing 100 µg/ml G418 and 50 µg/ml hygromycin B, and the culture was continued under the same conditions to obtain a cell line in which retrovirus vectors LXSN-16E7 and LXSH-hTERT have been incorporated.

The cell line thus obtained was further infected with a retrovirus vector MSCVpuro-16E6SDD151 containing a 16E6SDD151 gene, which is a deletion mutant in which HPV16-derived E6 gene transformation activity had been deleted. Specifically, the cell line was grown in the same medium containing 100 µg/ml G418 and 50 µg/ml hygromycin B, and, on the day before infection of 5 × 10⁶ cells with virus, was inoculated on a 6-cm culture dish and was cultured in an incubator under conditions of 37°C and CO₂ concentration 5% overnight. Polybrene was added to a final concentration of 8 µg/ml to a solution containing a retrovirus vector MSCVpuro-16E6SDD151, which expresses a 16E6SDD151 gene, prepared by the method described in a document [J. Gene Med., 6, 833-845 (2004)]. The supernatant (2 ml) of the culture of the human endometrium-derived cell line into which an HPV16-derived E7 gene and hTERT had been introduced, was replaced with 2 ml of the virus liquid, followed by culture in an incubator under conditions of 37°C and CO₂ concentration 5% overnight. The medium was replaced with the same medium containing 100 µg/ml G418, 50 µg/ml hygromycin B, and 1 µg/ml puromycin, and the culture was continued under the same conditions to obtain a cell line in which retrovirus vectors LXSN-16E7, LXSH-HTERT and MSCVpuro-16E6SDD151 have been incorporated.
Culture was continued under the above conditions for about four months, and immortalized cells were selected, followed by dilution to establish two types of independent single cell-derived cell lines.

### (3) Induction of cardiac muscle cells from human endometrium-derived immortalized cell lines

The capability of human endometrium-derived immortalized cell lines to be differentiated into cardiac muscle cells has been studied as follows.
The two types of human endometrium-derived immortalized cell lines obtained in the above (2) were infected with an adenovirus which expresses a green fluorescent protein (hereinafter abbreviated to GFP) according to the method described in a document [J. Gene Med., 6, 833-845 (04)]. Specifically, the human endometrium-derived immortalized cell line was inoculated at a density of 5 × 10⁴/cm² in a DMEM medium containing 10% FCS in a 6-cm culture dish and, on the next day, was infected with an adenovirus which expresses GFP. The supernatant was removed followed by further culture in the same medium in an incubator under conditions of 37°C and CO₂ concentration 5%. The expression of GFP was confirmed under a fluorescent confocal microscope.

For a 5-azacytidine (hereinafter abbreviated to 5-aza-C; manufactured by Sigma) treatment group, the GFP-labeled human endometrium-derived immortalized cell line was cultured for demethylation using a 10% FCS-containing DMEM medium, to which 5-aza-C had been added to a concentration of 3 µmol/l, in an incubator under conditions of CO₂ concentration 5% and 37°C for 24 hr. 5-aza-C was then removed by medium replacement, and, one day after that, the cells were harvested and were divided into cells to be solely cultured and cells to be cocultured with mouse fetus-derived cardiac muscle cells. On the other hand, for a 5-aza-C untreatment group, culture was carried out under the same conditions as described above except that the medium was free from 5-aza-C. The cells were then harvested and were divided into cells to be solely cultured and cells to be cocultured with mouse fetus-derived cardiac muscle cells.

The coculture with mouse fetus-derived cardiac muscle cells was carried out under conditions described in a document [J. Gene Med., 6, 833-845 (04)]. Specifically, the 5-aza-C treated human endometrium-derived immortalized cell line and 5-aza-C untreated human endometrium-derived immortalized cell line were peeled off from the culture dish by PBS (phosphate-buffered saline) containing 0.05% trypsin (manufactured by GIBCO) and 0.25 mmol/l EDTA (ethylenediamine-N,N,N',N'-tetraacetic acid) and were overlaid onto mouse fetus-derived cardiac muscle cells which had been previously inoculated at a density of 5 × 10³/cm². The human endometrium-derived immortalized cell line was induced into cardiac muscle cells by culture in a 10% FCS-containing DMEM medium in an incubator under conditions of CO₂ concentration 5% and 37°C. The human endometrium-derived immortalized cell line not to be cocultured was likewise peeled off from the culture dish by PBS containing 0.05% trypsin and 0.25 mmol/l EDTA and was then cultured in a 10% FCS-containing DMEM medium in an incubator under conditions of CO₂ concentration 5% and 37°C.

For the culture dish for the two types of human endometrium-derived immortalized cell lines which had been cocultured with mouse fetus-derived cardiac muscle cells for one week, visual observation showed that substantially 100% autonomous beating occurred and GFP fluorescence-emitting cells were contracted synchronously with each other. Since, however, all the cells were synchronized, the assay of the proportion of the cells which accurately caused cardiac muscle induction was difficult. Accordingly, for the two types of human endometrium-derived immortalized cell lines, a sample, which had been cocultured with mouse fetus-derived cardiac muscle cells for one week, and a sample, which had been solely cultured without coculture, were provided. In order to remove the serum in the culture, the sample was washed once with PBS. PBS containing 0.05% trypsin and 0.25 mmol/l EDTA was then added thereto, and the mixture was enzymatically reacted in a humidified incubator of 37°C for 5 min. The cells were peeled off as a lump from the Petri culture dish. The peeled tissue was placed in PBS containing 0.5% collagenase (Typell, manufactured by Worthington) and 10 mmol/l 2,3-butanedione monoxime (hereinafter abbreviated to BDM), and a reaction was further allowed to proceed for additional 20 min in a thermostatic chamber of 37°C, and cells were isolated. Since these cells contain mouse-derived cells, cell isolation was carried out using an anti-human-CD59 antibody (BD-555764, manufactured by Becton Dickinson Bioscience), which is an antibody to the antigen expressed in the human endometrium-derived immortalized cell line, and magnetic microbeads (BD IMagnet Cell Separation Magnet, BD-552311, manufactured by Becton Dickinson Bioscience). Upon the isolation of the cells using the anti-human-CD59 antibody, GFP-negative cells were not included, and substantially only human-derived cells were harvested.

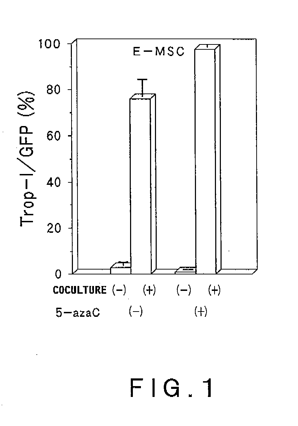

For the cells, staining was carried out using an antibody (Monoclonal mouse anti-cardiac Troponin I for human Catalogue # 4T21, manufactured by HyTest) to Troponin I which is an intracellular antigen and is a human cardiac muscle-specific protein. At the outset, a slide glass on its surface was coated with poly-L-lysine (P8920, manufactured by Sigma). The slide glass was previously washed with 1% HCl-containing 70% ethanol, and poly-L-lysine diluted with pure water by a factor of 10 was added thereto, followed by standing at room temperature for 5 min. Thereafter, poly-L-lysine was removed, and the slide glass was thoroughly dried at 60°C for one hr. A suspension of the human endometrium-derived immortalized cell line was placed on the lysine-coated slide glass for one hr to strongly bond the cell line to the slide glass. Thereafter, the cells were fixed with 4% paraformaldehyde, followed by immersion in PBS containing 0.02% Triton-X for 20 min to destroy cell membranes. A solution prepared by diluting an anti-human cardiac muscle Troponin I antibody as a primary antibody with PBS by a factor of 400 was then added thereto, and a reaction was allowed to proceed with moisture keeping in a refrigerator for one day, followed by washing with PBS ten times to remove the residual antibody. A secondary antibody (Anti Mouse TRITC, manufactured by Sigma) solution was added thereto, and a reaction was allowed to proceed with moisture keeping at room temperature for 30 min. The sample was observed under a confocal laser microscope (FV1000, manufactured by Olympus). As a result, it was found that an image of human cardiac muscle Troponin I which had been stained in a donut form while avoiding the nucleus in its center, was observed. This was determined to be positive, and the proportion of Troponin I expressed cells in the GFP-positive cells was calculated as a cardiomyogenic differentiation ratio. The results were as shown in Fig. 1.

As a result, regardless of the 5-aza-C treatment, the proportion of the Troponin I-positive cells was very low for the two types of human endometrium-derived immortalized cell lines which had been solely cultured without coculture with the mouse fetus-derived cardiac muscle cells (not more than 5% of the GFP-positive cells). On the other hand, for the case where coculture with mouse fetus-derived cardiac muscle cells, the proportion of Troponin I-positive cells was substantially 100% for the two types of human endometrium-derived immortalized cell lines which had been treated with 5-aza-C and was about 80% even for the two types of human endometrium-derived immortalized cell lines which had not been subjected to 5-aza-C treatment which had hitherto been considered indispensable for cardiac muscle induction. The above results demonstrate that, as compared with the proportion of induction into human cardiac muscle cells in the prior art technique (generally not more than 1%), the human endometrium-derived immortalized cell lines, when cocultured with cardiac muscle cells, cause autonomous beating at a very high percentage and are already induced into cardiac muscles having a physiological function on the culture dish, regardless of the 5-aza-C treatment.

The two types of cells thus obtained were designated as E-MSC (hEPC100) cells and E-MSC (hEPC214) cells. When both the types of cells are collectively referred to in the following description, they will be collectively referred to as a human endometrium-derived immortalized cell line or E-MSC.

### (4) Coculture involving isolation with collagen film

The following experiment was carried out to substantiate that the high cardiomyogenic differentiation ratio in the above (3) was not the result of cell fusion between mouse fetus-derived cardiac muscle cells and human endometrium-derived immortalized cell line. Specifically, a collagen film for tissue culture (CM-6, manufactured by Koken) was provided. Mouse fetus-derived cardiac muscle cells were cultured on the upper surface the collagen film. E-MSC was cultured on the lower surface, and the film was observed. Cell density, culture conditions and the like were basically the same as those described above. Specifically, mouse fetus-derived cardiac muscle cells were inoculated at a density of 1 × 10⁵ cells /cm² on the upper surface of the collagen film for tissue culture. The medium was a 10% FCS-containing DMEM medium, and the culture was carried out in an incubator under conditions of CO₂ concentration 5% and 37°C for 24 hr. On the other hand, the human endometrium-derived immortalized cell line was inoculated at a density of 5 × 10⁴/cm² in a 10% FCS-containing DMEM medium in a 6-cm culture dish and, on the next day, was infected with an adenovirus which expresses GFP. The supernatant was removed followed by further culture in the same medium in an incubator under conditions of 37°C and CO₂ concentration 5%. The expression of GFP was confirmed by observation under a fluorescent confocal microscope. The collagen film in which the mouse fetus-derived cardiac muscle cells were cultured on its upper surface was turned upside down so that the bottom face of the collagen film face upward. The GFP-labeled human endometrium-derived immortalized cell line collected one day after the infection was inoculated at a density of 5 × 10⁴/cm² on the bottom face of the collagen film, followed by culture in a 10% FCS-containing DMEM medium in an incubator under conditions of CO₂ concentration 5% and 37°C for 24 hr. The collagen film was transferred to another culture dish around the time when the cells were satisfactorily adhered, and the culture was again continued in such a state that the mouse fetus-derived cardiac muscle cells were located on the upper surface. Under the above culture conditions, since E-MSC was physically completely isolated from the primary mouse cultured cardiac muscle cells by the collagen film and thus was not physically in contact with the primary mouse cultured cardiac muscle cells. Therefore, cell fusion does not occur. Under the above culture conditions, autonomous beating of E-MSC began one week after the start of the culture. E-MSC was observed under a confocal laser microscope. As a result, the expression of human Troponin I could be clearly confirmed, substantiating that cardiac muscle induction of E-MSC was not due to cell fusion.

### Example 2

### Properties of cardiac muscle cells induced from E-MSC

On the second day after the start of coculture with mouse fetus-derived cardiac muscle cells, E-MSC started autonomous beating, and, on the third to fourth day after the start of the coculture, E-MSCs were synchronized with each other and started contraction. It could be visually observed that 100% of GFP fluorescence emitting cells caused contraction. In this state, culture could be continued for about one month. E-MSC was not treated with 5-aza-C and was induced into cardiac muscles by only coculture with mouse fetus-derived cardiac muscle cells. A cardiac muscle-specific protein mRNA expressed in E-MSC induced into cardiac muscles under such conditions was measured by RT-PCR. On the seventh day after the start of the coculture, the cells were washed with PBS once to remove the serum contained in the culture. Thereafter, PBS containing 0.05% trypsin and 0.25 mmol/l EDTA was added, and the mixture was enzymatically reacted in a humidified incubator at 37°C for 5 min. The cells were peeled off as a lump from the Petri culture dish. The peeled tissue was placed in PBS containing 0.5% collagenase and 10 mmol/l BDM, and the mixture was allowed to react in a thermostat chamber of 37°C for 20 min, followed by isolation of cells. The isolated cells were subjected to magnetic bead cell isolation using an anti-human-CD59 antibody for human-derived cell enrichment. The whole RNA was extracted from the enriched cells based on a document [J. Gene Med., 6, 833-845 (04)], and first-strand cDNA was synthesized. Specifically, the whole RNA was extracted from the cells using Isogen (manufactured by NIPPON GENE CO., LTD.), and cDNA was synthesized using a first-strand cDNA synthesis kit (manufactured by Amersham Pharmacia Biotec). Commercially available cardiac muscle-derived RNA was purchased from Clontech and was converted to cDNA in the same manner as described above.

Next, in order to study the expression of a cardiac muscle cell-specific gene, quantitative PCR was carried out using first-strand cDNA as a substrate and synthetic DNA having a base sequence shown in SEQ ID Nos. 1 to 36. ANP and BNP as natriuretic peptides, Troponin T and Troponin I as proteins which function to regulate muscle contraction, cardiac actin as an actin, MLC-2a as a myosin light chain, and Nkx2.5/Csx and GATA4 as cardiac muscle cell-specific transcription factors were used as cardiac muscle cell-specific genes. Since these sequences were set as a human-specific primer, the inclusion of the mouse cardiac muscle cell-derived cDNA in the sample does not have any influence on measurement results.

Synthetic DNA having a base sequence shown in SEQ ID Nos. 1 and 2 was used for the amplification of ANP; synthetic DNA having a base sequence shown in SEQ ID Nos. 3 and 4 was used for the amplification of BNP; synthetic DNA having a base sequence shown in SEQ ID Nos. 5 and 6 was used for the amplification of Troponin T; synthetic DNA having a base sequence shown in SEQ ID Nos. 7 and 8 was used for the amplification of Troponin I; synthetic DNA having a base sequence shown in SEQ ID Nos. 9 and 10 was used for the amplification of cardiac actin; synthetic DNA having a base sequence shown in SEQ ID Nos. 11 and 12 was used for the amplification of MLC-2a; synthetic DNA having a base sequence shown in SEQ ID Nos. 13 and 14 was used for the amplification of Nkx2.5/Csx as cardiac muscle cell-specific transcription factors, and synthetic DNA having a base sequence shown in SEQ ID Nos. 15 and 16 was used for the amplification of GATA4.

As a result of RT-PCR analysis, as with the human cardiac muscle cells, in the E-MSC cocultured with mouse fetus-derived cardiac muscle cells, it was observed that mRNA of ANP and BNP as natriuretic peptides, Troponin T and Troponin I as proteins which function to regulate muscle contraction, cardiac actin as an actin, MLC-2a as a myosin light chain, and Nkx2.5/Csx and GATA4 as cardiac muscle cell-specific transcription factors was expressed as cardiac muscle cell-specific genes.

It was observed that, in E-MSC, these cardiac muscle cell-specific gene mRNAs were expressed from before the coculture. The fact that gene introduction through immortalization does not cause a change in properties of mesenchymal cells per se [J. Gene Med., 6, 833-845 (04)], suggests the presence of a large quantity of mesenchymal stem cells, which may call such cardiac muscle ancestor cells, in the endometrium.

In the observation under a confocal laser microscope, E-MSC differentiated into cardiac muscle cells strongly expressed human cardiac muscle cell-specific Troponin I, and a fiber-like structure characteristic of cells which cause strong contraction could be observed. A cardiac muscle Troponin I reaction was detected only in E-MSC which emits green GFP fluorescence.

Studies on α-actinin staining of E-MSC differentiated into cardiac muscle cells, and immunostaining of a connexin 43 (hereinafter abbreviated to Cx43) protein indispensable for electrical bonding of cardiac muscle to each other and cooperative contraction of the cardiac muscles were carried out with a confocal laser microscope. A clear and developed striation structure having a given oriented structure could be observed by α-actinin staining. This means that the present cells contracted very strongly, and is in agreement with the results of macroscopic observation under a microscope.

Further, a very strong stained image Cx43 surrounding the margin of the cytoplasm with the nucleus located as the center was observed. This fact indicates that the present cells have been electrically strongly bonded to the surrounding cells, clearly demonstrating a synchronous phenomenon by the automaticity of the present cells observed upon the contraction.

Cells which cause spontaneous excitation contraction are not limited to cardiac muscle cells. Skeletal muscle and smooth muscle cells as well sometimes cause spontaneous excitation. Accordingly, in order to demonstrate that the present cells are cardiac muscle cells, the action potential should be measured. The action potential of the cardiac muscle cells has a specific plateau phase caused by an L-type Ca current specific for cardiac muscles, and, thus, the action potential duration has a width of about 200 to 300 msec.

Two types of E-MSC were induced into cardiac muscles by only coculture with mouse fetus-derived cardiac muscle cells without 5-aza-C treatment, and the action potential was recorded from the E-MSC induced into cardiac muscles. Since E-MSC is present on the mouse fetus-derived cardiac muscle cells, when a recording electrode is inserted into the E-MSC cell, there is a possibility that the electrode is inserted into mouse cardiac muscle cells located just thereunder. Accordingly, the action potential was measured based on the method described in a document [J. Gene Med., 6, 833-845 (04)]. Specifically, 0.5 mmol/l Alexa-568 was dissolved in a 2 mol/l internal fluid of KCl electrode, and the solution was sealed into a recording glass electrode followed by recording. That is, a microelectrode for intracellular potential recording, having a resistance value of about 10 to 20 MΩ in such a state that 2 mol/l KCI has been sealed, was prepared from a glass tube (CG-150-100TF, manufactured by WPI) using an electrode puller P-97 manufactured by Sutter. The cells were kept and held at 37°C on a noiseless heat plate (U-PHP, manufactured by Olympus) on a magnetizing specification gibraltar platform (Gibraltar XX-1ss) installed on a vibration removing table (AZI-0806HS, manufactured by MEIRITSU SEIKI CO., LTD.). An experiment was carried out while observation of the cells under an inverted epifluorescence microscope (IX-70-23FL/DIC, manufactured by Olympus) Nomarski differential interference microscope. Autonomous beating cells which emit green fluorescence were visually identified by observation under the epifluorescence microscope. The cells were accessed to the glass electrode using a piezo drive-type patch clamp micromanipulator (PCS-5400, manufactured by Narishige), and the glass electrode was inserted thereinto. Specifically, the tip of the electrode was adhered to the surface of the cell membrane with caution so as not to be pierced through the cell. A hole was formed in the cell membrane with a buzz system, and the electrode was inserted into the cell. Recording could be stably carried out for 5 to 10 min. Since the tip of the electrode was small, a red coloring matter could not be substantially observed within the cell during potential recording. After the completion of potential recording, however, upon the flow of a current of about 10 nA into the cell through the electrode, the charged Alexa-568 was penetrated into the cell by ionophoresis, resulting in rapid staining of the whole cell. A comparison of the form of the cell which emit green fluorescence with a red staining pattern observed after recording of the potential shows that the cell of which the potential has been recorded is a GFP-positive E-MSC cell. Regarding the potential, the intracellular potential was amplified through a patch clamp amplifier (Axopatch 200B, manufactured by Axon) through a platinum electrode inserted into the glass electrode, and the waveform was introduced through a data acquisition device (pClamp8) on a computer.

The recorded action potential has a pacemaker potential characteristic of cells having automaticity, and one E-MSC exhibited a sinoatrial action potential which is a relatively short action potential while another E-MSC exhibited a working myocardium-type action potential which has a broad action potential width and is deep in resting membrane potential. Both the E-MSCs had a cardiac muscle-specific potential waveform.

The above results show that, upon coculture with mouse fetus-derived cardiac muscle cells, E-MSC is differentiated into working myocardium-type cardiac muscle cells and sinoatrial node cell-type cardiac muscle cells.

### Example 3

### Analysis of surface antigen of E-MSC

In order to efficiently isolate cells having cardiac myogenesis from a human endometrium and to purify the isolated cells, the surface antigen of E-MSC cells was analyzed with a flow cytometer (BD FACSort, manufactured by Becton Dickinson Bioscience).
In the analysis, the following 16 types of antigens were used: Flk-1 and CD31 known as surface antigens of vascular endothelial cells; CD13, CD14, CD34, CD45, and CD90 known as surface antigens of hematopoietic cells; CD140a (PDGF receptor α; hereinafter abbreviated to PDGFRα) known as a surface antigen of mesenchymal cells; integrin CD29; matrix receptors CD44, CD50, and CD54; CD24 as a marker of B cells and granulocytes; CD55 and CD59 widely expressed in blood cells; and adhesion molecule CD166 which is a member of an immunoglobulin superfamily.

Each antibody was added in a concentration of 2 µg/5 × 10⁵ cells/0.2 ml to endometrial cells suspended in a buffer solution for FACS (1% FCS-PBS), and a reaction was allowed to proceed in ice for 30 min.

Cells which had been reacted only with an anti-mouse IgTRITC-labeled antibody (AP160R, manufactured by Chemicon), were used as a negative control.
The cells treated with the FITC-labeled or PE-labeled antibody were washed thrice with a buffer solution and were then suspended in 0.5 ml thereof.
The cells treated with the unlabeled primary antibody were washed with a buffer solution and were reacted with the anti-mouse IgTRITC-labeled antibody (concentration: 2 µg/5 × 10⁵ cells/0.2 ml) in ice for 30 min. The cells were then washed thrice with a buffer solution and were suspended in 0.5 ml thereof.
The fluorescence intensity was measured with a flow cytometer (BD FACSort, manufactured by Becton Dickinson Bioscience), and whether or not the antigen molecule was expressed was determined based on whether or not the fluorescence intensity was increased.
Regarding the expression of the CD140a antigen, an antibody reaction was carried out using an unlabeled anti-human CD140a (PDGFRα) antibody (556001, manufactured by PharMingen), and the reaction product was treated with an anti-mouse IgTRITC-labeled antibody, followed by measurement with a flow cytometer.

For the expression of the Flk-1 antigen, an antibody reaction was carried out with an unlabeled anti-human Flk-1 antibody (sc-6251, manufactured by Santa Cruz), and the reaction product was treated with an anti-mouse IgTRITC-labeled antibody, followed by measurement with a flow cytometer.

For the expression of the CD31 antigen, an antibody reaction was carried out with an FITC-labeled anti-human CD31 antibody (IM1431, manufactured by Beckman Coulter) followed by measurement with a flow cytometer.

For the expression of the CD13 antigen, an antibody reaction was carried out with an FITC-labeled anti-human CD13 antibody (IM0778, manufactured by Beckman Coulter) followed by measurement with a flow cytometer.

For the expression of the CD14 antigen, an antibody reaction was carried out with an FITC-labeled anti-human CD14 antibody (PN6604110, manufactured by Beckman Coulter) followed by measurement with a flow cytometer.

For the expression of the CD34 antigen, an antibody reaction was carried out with an PE-labeled anti-human CD34 antibody (IM1250, manufactured by Beckman Coulter) followed by measurement with a flow cytometer.

For the expression of the CD45 antigen, an antibody reaction was carried out with an FITC-labeled anti-human CD45 antibody (PM31254X, manufactured by PharMingen) followed by measurement with a flow cytometer.

For the expression of the CD90 antigen, an antibody reaction was carried out with an PE-labeled anti-human CD90 antibody (HD7403, manufactured by Beckman Coulter) followed by measurement with a flow cytometer.

For the expression of the CD29 antigen, an antibody reaction was carried out with an FITC-labeled human CD29 antibody (PN6604105, manufactured by Beckman Coulter) followed by measurement with a flow cytometer.

For the expression of the CD44 antigen, an antibody reaction was carried out with an FITC-labeled human CD44 antibody (IM1219, manufactured by Beckman Coulter) followed by measurement with a flow cytometer.

For the expression of the CD50 antigen, an antibody reaction was carried out with an PE-labeled anti-human CD50 antibody (IM1601, manufactured by Beckman Coulter) followed by measurement with a flow cytometer.

For the expression of the CD54 antigen, an antibody reaction was carried out with an unlabeled human CD54 antibody (IM0544, manufactured by Beckman Coulter), and the reaction product was treated with an anti-mouse IgTRITC-labeled antibody followed by measurement with a flow cytometer.

For the expression of the CD24 antigen, an antibody reaction was carried out with an unlabeled human CD24 antibody (IM0118, manufactured by Beckman Coulter), and the reaction product was treated with an anti-mouse IgTRITC-labeled antibody followed by measurement with a flow cytometer.

For the expression of the CD55 antigen, an antibody reaction was carried out with an FITC-labeled anti-human CD55 antibody (IM2725, manufactured by Beckman Coulter) followed by measurement with a flow cytometer.

For the expression of the CD59 antigen, an antibody reaction was carried out with an FITC-labeled anti-human CD59 antibody (IM3457, manufactured by Beckman Coulter) followed by measurement with a flow cytometer.

For the expression of the CD166 antigen, an antibody reaction was carried out with an unlabeled anti-human CD166 antibody (RDI-CD166-aA6, manufactured by RDI) followed by measurement with a flow cytometer.

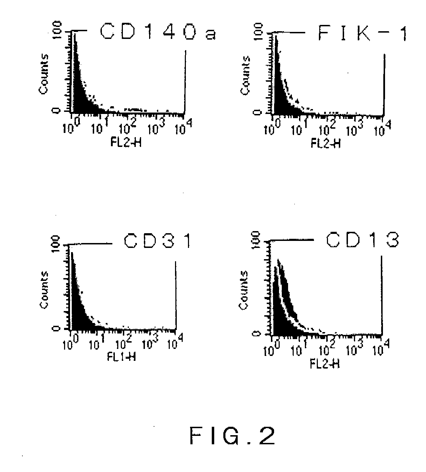

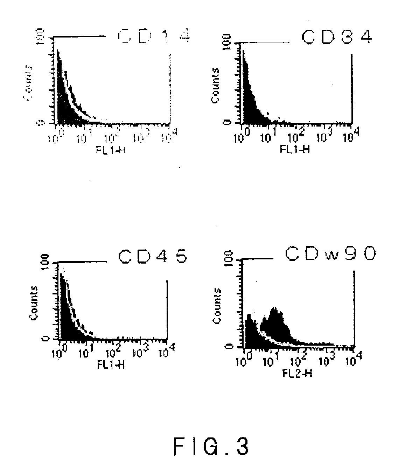

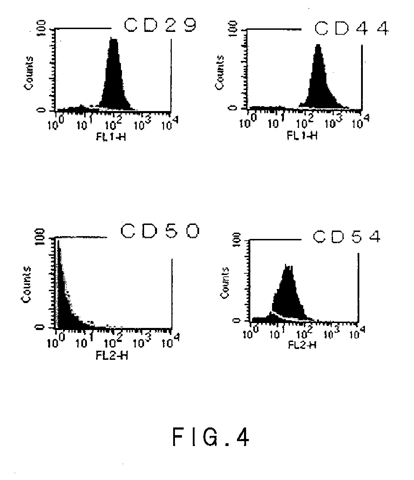

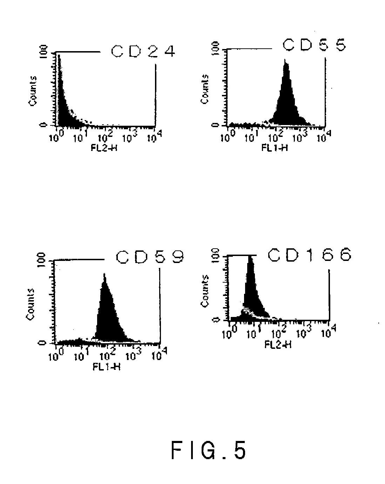

The results of the measurement with the flow cytometer were as shown in Figs. 2 to 5. The results of the analysis are summarized in the following table.

**[Table 1]**

| surface molecule | E-MSC |
|---|---|
| CD13 | + - +/- |
| CD14 | - |
| CD24 | - |
| CD29 | + |
| CD31 (PECAM-1) | - |
| CD34 | - |
| CD44 (Hyaluronate) | + |
| CD45 | - |
| CD50 (ICAM-3) | - |
| CD54 (ICAM-1) | + - +/- |
| CD55 (DAF) | + |
| CD59 | + |
| CD90 (Thy-1) | + - +/- |
| CD140a (PDGFRα) | - |
| CD166(ALCAM) | + |
| Flk-1 | - |

### Example 4

### In vivo transplantation experiment

An E-GFP-stained sheet-like transplantation graft was obtained in the same manner as in Example 2 (4). The sheets thus obtained were overlaid on top of each other in a culture to form an assembly of two or three layers, and a collagen film (CM-6, manufactured by Kohken). The collagen film was transplanted on the surface of the heart of a nude rat free from immunologic rejection, and the chest was closed. Two weeks after the transplantation, the chest was reopened. The heart was fixed, and immunostaining was carried out. The heart was stained with an anti-Cardiac Troponin-I antibody, a Sarcomeric alpha actinin antibody, and a Connexin 43 antibody, and whether or not the transplantation graft was induced into cardiac muscles was confirmed with a confocal laser microscope. As a result, a clear striation specific for cardiac muscles, clear staining of Cardiac troponin-I, and the expression of Connexin43 protein were observed. Thus, it was confirmed that the transplanted graft was introduced in the transplanted site into cardiac muscles at a high percentage to form full-thickness cardiac muscles.

### Example 5

### (1) Isolation of cells from human menstrual blood and culture of the cells

A menstrual blood on the first day of a menstrual period (the day of the start of a menstrual period) was transferred through a sanitary product or directly from an genital area to a 10-ml sterile plastic dish. The menstrual blood harvested in the plastic dish was moved to a 50-ml plastic tube containing 20 ml of a culture medium [1% fetal bovine serum-containing DMEM (Dulbecco's modified MEM) medium; penicillin/streptomycin antibiotic added]. The culture containing the acquired menstrual blood was centrifuged at 1,000 × g to collect cells contained in the menstrual blood. The menstrual blood cells were resuspended in an α-MEM (α-modified MEM) medium containing 10% FBS (fetal bovine serum) to a menstrual blood-derived cell fluid.

The isolated cells contained in the menstrual blood were dispersed in an α-MEM medium containing 10 to 20% of a bovine serum or the like. The dispersion was placed in a plastic culture dish for tissue culture and was cultured in an incubator filled with 5 to 10% carbon dioxide gas at a culture temperature of 33 to 37°C. On the second day from the start of culture, medium replacement was carried out for the removal of erythrocytes. The medium was removed around the time when the cells were grown over the whole area of the culture dish. A trypsin EDTA solution was added to suspend the cells.

### (2) Cardiac muscle induction from human menstrual blood mesenchymal stem cells

The differentiation capability of the human menstrual blood mesenchymal stem cells obtained in the above (1) into cardiac muscle cells was studied as follows. The human menstrual blood mesenchymal stem cells were infected with an adenovirus which expresses GFP according to the method described in a document [J. Gene Med., 6, 833-845 (04)]. Specifically, the human menstrual blood mesenchymal stem cells were inoculated at a density of 5 × 10⁴/cm² in a DMEM medium containing 10% FCS in a 6-cm culture dish and, on the next day, were infected with an adenovirus which expresses GFP. The supernatant was removed followed by further culture in an incubator under conditions of 37°C and CO₂ concentration 5%. The expression of GFP was confirmed under a fluorescent confocal microscope.

The coculture with mouse fetus-derived cardiac muscle cells was carried out under conditions described in a document [J. Gene Med., 6, 833-845 (04)]. Specifically, the 5-aza-C untreated mesenchymal stem cells derived from human placenta and umbilical cord were peeled off from the culture dish by PBS (phosphate-buffered saline) containing 0.05% trypsin (manufactured by GIBCO) and 0.25 mmol/l EDTA (ethylenediamine-N,N,N',N'-tetraacetic acid) and were overlaid onto mouse fetus-derived cardiac muscle cells which had been previously inoculated at a density of 5 × 10³/cm². The mesenchymal stem cells derived from human placenta and umbilical cord were induced into cardiac muscle cells by culture in a 10% FCS-containing DMEM medium in an incubator under conditions of CO₂ concentration 5% and 37°C. The mesenchymal stem cells derived from human placenta and umbilical cord not to be cocultured were likewise peeled off from the culture dish by PBS containing 0.05% trypsin and 0.25 mmol/l EDTA and was then cultured in a 10% FCS-containing DMEM medium in an incubator under conditions of CO₂ concentration 5% and 37°C.

### (3) Percentage induction of human menstrual blood mesenchymal stem cells into cardiac muscles

As a result of visual observation, it was found that, for the culture dish containing human menstrual blood mesenchymal stem cells cocultured with mouse fetus-derived cardiac muscle cells for one week, about 50% of the cells exhibited autonomous beating, and the cells which emit GFP fluorescence were synchronized with each other and were contracted. Since, however, all the cells are synchronized, it was difficult to accurately evaluate the proportion of cells which cause cardiac muscle induction. Accordingly, for the human menstrual blood mesenchymal stem cells, a sample, which had been cocultured with mouse fetus-derived cardiac muscle cells for one week, and a sample, which had been solely cultured without coculture, were provided. In order to remove the serum in the culture, the sample was washed once with PBS. PBS containing 0.05% trypsin and 0.25 mmol/l EDTA was then added thereto, and the mixture was enzymatically reacted in a humidified incubator of 37°C for 5 min. The cells were peeled off as a lump from the Petri culture dish. The peeled tissue was placed in PBS containing 0.5% collagenase (Typell, manufactured by Worthington) and 10 mmol/l 2,3-butanedione monoxime (hereinafter abbreviated to BDM), and a reaction was further allowed to proceed for additional 20 min in a thermostatic chamber of 37°C, and cells were isolated.

For the cells, staining was carried out using an antibody (Monoclonal mouse anti-cardiac Troponin I for human Catalogue # 4T21, manufactured by HyTest) to Troponin I which is an intracellular antigen and is a human cardiac muscle-specific protein. At the outset, a slide glass on its surface was coated with poly-L-lysine (P8920, manufactured by Sigma). The slide glass was previously washed with 1% HCl-containing 70% ethanol, and poly-L-lysine diluted with pure water by a factor of 10 was added thereto, followed by standing at room temperature for 5 min. Thereafter, poly-L-lysine was removed, and the slide glass was thoroughly dried at 60°C for one hr. A suspension of the human menstrual blood mesenchymal stem cells was placed on the lysine-coated slide glass for one hr to strongly bond the cells to the slide glass. Thereafter, the cells were fixed with 4% paraformaldehyde, followed by immersion in PBS containing 0.02% Triton-X for 20 min to destroy cell membranes. A solution prepared by diluting an anti-human cardiac muscle Troponin I antibody as a primary antibody with PBS by a factor of 400 was then added thereto, and a reaction was allowed to proceed with moisture keeping in a refrigerator for one day, followed by washing with PBS ten times to remove the residual antibody. A secondary antibody (Anti Mouse TRITC, manufactured by Sigma) solution was added thereto, and a reaction was allowed to proceed with moisture keeping at room temperature for 30 min. The sample was observed under a confocal laser microscope (FV1000, manufactured by Olympus). As a result, it was found that an image of human cardiac muscle Troponin I which had been stained in a donut form while avoiding the nucleus in its center, was observed. This was determined to be positive, and the proportion of Troponin I expressed cells in the GFP-positive cells was calculated as a cardiomyogenic differentiation ratio. As a result, it was found that Troponin-I was expressed in about 30% of the human menstrual blood mesenchymal stem cells.

It was found that, regardless of the 5-aza-C treatment, the proportion of the Troponin I-positive cells was very low for the human menstrual blood mesenchymal stem cells which had been solely cultured without coculture with the mouse fetus-derived cardiac muscle cells (not more than 1% of the GFP-positive cells). On the other hand, for the case where coculture with mouse fetus-derived cardiac muscle cells was carried out, the proportion of Troponin I-positive cells was substantially 30% even for the human menstrual blood mesenchymal stem cells which had not been subjected to 5-aza-C treatment which had hitherto been considered indispensable for cardiac muscle induction. The above results demonstrate that, as compared with the proportion of induction into human cardiac muscle cells in the prior art technique (generally not more than 1%), the human menstrual blood mesenchymal stem cells, which, when cocultured with cardiac muscle cells, cause autonomous beating at a very high percentage and are already induced into cardiac muscles having a physiological function on the culture dish, regardless of the 5-aza-C treatment.

### (4) Cardiac muscle-specific action potential obtained from human menstrual blood mesenchymal stem cells

The action potential was measured based on the method described in a document [J. Gene Med., 6, 833-845 (04)] in the same manner as in Example 2. As a result, the recorded action potential was that characteristic of cardiac muscles having a width of about 300 msec and had a diastolic slow depolarization (pacemaker potential).

### Example 6

### (1) Induction of mesenchymal stem cells derived from human placenta and umbilical cord into cardiac muscles

Studies on the capability of mesenchymal stem cells derived from human placenta and umbilical cord to be differentiated into cardiac muscle cells were made as follows.
The placenta and umbilical cord dropped from the uterus at the time of birth were harvested and were moved to a 50-ml plastic tube containing 20 ml of a culture medium [1% fetal bovine serum-containing DMEM (Dulbecco's modified MEM) medium; penicillin/streptomycin antibiotic and heparin (100 U) added]. The tissue was lightly washed within the tube and was transferred to a 10-ml sterile plastic dish. The tissue was chopped (size: about 1 to 5 mm³) with an ophthalmic scissors within the plastic dish, and the chopped tissue was allowed to stand for a few min. The supernatant containing blood cells was discarded, and the tissue was again washed with DMEM. This procedure was repeated three or four times. The tissue was then resuspended in an α-MEM (α-modified MEM) medium containing 10% FBS (fetal bovine serum).

The liquid containing placenta and umbilical cord tissues was dispersed in an α-MEM medium containing 10 to 20% of bovine or other serum. The dispersion liquid was placed in a plastic culture dish for tissue culture and was cultured at a culture temperature of 33 to 37°C in an incubator filled with 5 to 10% carbon dioxide gas. On the second day after the start of the culture, the medium was replaced to remove erythrocytes. Around the time when the cells were grown over the whole area of the culture dish, the medium was removed and a trypsin EDTA solution was added to suspend the cells.
The mesenchymal stem cells derived from human placenta and umbilical cord thus obtained were infected with an adenovirus which expresses GFP according to the method described in a document [J. Gene Med., 6, 833-845 (04)]. Specifically, the mesenchymal stem cells derived from human placenta and umbilical cord were inoculated at a density of 5 × 10⁴/cm² in a DMEM medium containing 10% FCS in a 6-cm culture dish, and, on the next day, were infected with an adenovirus which expresses GFP. The supernatant was removed followed by further culture in the same medium in an incubator under conditions of 37°C and CO₂ concentration 5%. The expression of GFP was confirmed with a fluorescent confocal microscope.

The coculture with mouse fetus-derived cardiac muscle cells was carried out under conditions described in a document [J. Gene Med., 6, 833-845 (04)]. Specifically, 5-aza-C untreated mesenchymal stem cells derived from human placenta and umbilical cord were peeled off from the culture dish by PBS (phosphate-buffered saline) containing 0.05% trypsin (manufactured by GIBCO) and 0.25 mmol/l EDTA (ethylenediamine-N,N,N',N'-tetraacetic acid) and were overlaid onto mouse fetus-derived cardiac muscle cells which had been previously inoculated at a density of 5 × 10³/cm². The mesenchymal stem cells derived from human placenta and umbilical cord were induced into cardiac muscle cells by culture in a 10% FCS-containing DMEM medium in an incubator under conditions of CO₂ concentration 5% and 37°C. The mesenchymal stem cells derived from human placenta and umbilical cord not to be cocultured were likewise peeled off from the culture dish by PBS containing 0.05% trypsin and 0.25 mmol/l EDTA and was then cultured in a 10% FCS-containing DMEM medium in an incubator under conditions of CO₂ concentration 5% and 37°C.

### (2) Cardiomyogenic differentiation ratio of mesenchymal stem cells derived from human placenta and umbilical cord

As a result of visual observation, it was found that, for the culture dish containing mesenchymal stem cells derived from human placenta and umbilical cord cocultured with mouse fetus-derived cardiac muscle cells for one week, about 30% of the cells caused automonous beating, and the cells which emit GFP fluorescence were synchronized with each other and were contracted. Since, however, all the cells are synchronized, it was difficult to accurately evaluate the proportion of cells which cause cardiac muscle induction. Accordingly, for the mesenchymal stem cells derived from human placenta and umbilical cord, a sample, which had been cocultured with mouse fetus-derived cardiac muscle cells for one week, and a sample, which had been solely cultured without coculture, were provided. In order to remove the serum in the culture, the sample was washed once with PBS. PBS containing 0.05% trypsin and 0.25 mmol/l EDTA was then added thereto, and the mixture was enzymatically reacted in a humidified incubator of 37°C for 5 min. The cells were peeled off as a lump from the Petri culture dish. The peeled tissue was placed in PBS containing 0.5% collagenase (Typell, manufactured by Worthington) and 10 mmol/l 2,3-butanedione monoxime (hereinafter abbreviated to BDM), and a reaction was further allowed to proceed for additional 20 min in a thermostatic chamber of 37°C, and cells were isolated.

For the cells, staining was carried out using an antibody (Monoclonal mouse anti-cardiac Troponin I for human Catalogue # 4T21, manufactured by HyTest) to Troponin I which is an intracellular antigen and is a human cardiac muscle-specific protein. At the outset, a slide glass on its surface was coated with poly-L-lysine (P8920, manufactured by Sigma). The slide glass was previously washed with 1% HCl-containing 70% ethanol, and poly-L-lysine diluted with pure water by a factor of 10 was added thereto, followed by standing at room temperature for 5 min. Thereafter, poly-L-lysine was removed, and the slide glass was thoroughly dried at 60°C for one hr. A suspension of the mesenchymal stem cells derived from human placenta and umbilical cord were placed on the lysine-coated slide glass for one hr to strongly bond the cell line to the slide glass. Thereafter, the cells were fixed with 4% paraformaldehyde, followed by immersion in PBS containing 0.02% Triton-X for 20 min to destroy cell membranes. A solution prepared by diluting an anti-human cardiac muscle Troponin I antibody as a primary antibody with PBS by a factor of 400 was then added thereto, and a reaction was allowed to proceed with moisture keeping in a refrigerator for one day, followed by washing with PBS ten times to remove the residual antibody. A secondary antibody (Anti Mouse TRITC, manufactured by Sigma) solution was added thereto, and a reaction was allowed to proceed with moisture keeping at room temperature for 30 min. The sample was observed under a confocal laser microscope (FV1000, manufactured by Olympus). As a result, it was found that an image of human cardiac muscle Troponin I which had been stained in a donut form while avoiding the nucleus in its center, was observed. This was determined to be positive, and the proportion of Troponin I expressed cells in the GFP-positive cells was calculated as a cardiomyogenic differentiation ratio. As a result, it was found that Troponin-I was expressed in about 20% of mesenchymal stem cells derived from human placenta and umbilical cord.

It was found that, regardless of the 5-aza-C treatment, the proportion of the Troponin I-positive cells was very low for the mesenchymal stem cells derived from human placenta and umbilical cord which had been solely cultured without coculture with the mouse fetus-derived cardiac muscle cells (not more than 1% of the GFP-positive cells). On the other hand, for the case where coculture with mouse fetus-derived cardiac muscle cells was carried out, the proportion of Troponin I-positive cells was about 20% even for the mesenchymal stem cells derived from human placenta and umbilical cord which had not been subjected to 5-aza-C treatment which had hitherto been considered indispensable for cardiac muscle induction. The above results demonstrate that, as compared with the proportion of induction into human cardiac muscle cells in the prior art technique (generally not more than 1%), the mesenchymal stem cells derived from human placenta and umbilical cord, which, when cocultured with cardiac muscle cells, cause autonomous beating at a very high percentage and are already induced into cardiac muscles having a physiological function on the culture dish, regardless of the 5-aza-C treatment.

### (2) Cardiac muscle-specific action potential obtained from mesenchymal stem cells derived from human placenta and umbilical cord

The action potential was measured based on the method described in a document [J. Gene Med., 6, 833-845 (04)] in the same manner as in Example 2. As a result, some of the recorded action potential was that characteristic of cardiac muscles having a width of about 300 msec and had a diastolic slow depolarization (pacemaker potential).

### Example 7

### (1) Induction of human amnion mesenchymal stem cells into cardiac muscles

Studies on the capability of human amnion mesenchymal stem cells to be differentiated into cardiac muscle cells were made as follows.
The human amnion mesenchymal stem cells prepared according to the method described in Example 6 (1) were infected with an adenovirus which expresses GFP according to the method described in a document [J. Gene Med., 6, 833-845 (04)]. Specifically, the human amnion mesenchymal stem cells were inoculated at a density of 5 × 10⁴/cm² in a DMEM medium containing 10% FCS in a 6-cm culture dish, and, on the next day, were infected with an adenovirus which expresses GFP. The supernatant was removed followed by further culture in the same medium in an incubator under conditions of 37°C and CO₂ concentration 5%. The expression of GFP was confirmed with a fluorescent confocal microscope.

The coculture with mouse fetus-derived cardiac muscle cells was carried out under conditions described in a document [J. Gene Med., 6, 833-845 (04)]. Specifically, 5-aza-C untreated human amnion mesenchymal stem cells were peeled off from the culture dish by PBS (phosphate-buffered saline) containing 0.05% trypsin (manufactured by GIBCO) and 0.25 mmol/l EDTA (ethylenediamine-N,N,N',N'-tetraacetic acid) and were overlaid onto mouse fetus-derived cardiac muscle cells which had been previously inoculated at a density of 5 × 10³/cm². The human amnion mesenchymal stem cells were induced into cardiac muscle cells by culture in a 10% FCS-containing DMEM medium in an incubator under conditions of CO₂ concentration 5% and 37°C. The human amnion mesenchymal stem cells not to be cocultured were likewise peeled off from the culture dish by PBS containing 0.05% trypsin and 0.25 mmol/l EDTA and was then cultured in a 10% FCS-containing DMEM medium in an incubator under conditions of CO₂ concentration 5% and 37°C.

### (2) Percentage induction of human amnion mesenchymal stem cells into cardiac muscles

As a result of visual observation, it was found that, for the culture dish containing human amnion mesenchymal stem cells cocultured with mouse fetus-derived cardiac muscle cells for one week, about 20% of the cells caused automonous beating, and the cells which emit GFP fluorescence were synchronized with each other and were contracted.

### (3) Cardiac muscle-specific action potential obtained from human amnion mesenchymal stem cells

The action potential was measured based on the method described in a document [J. Gene Med., 6, 833-845 (04)] in the same manner as in Example 2. As a result, the recorded action potential was that characteristic of cardiac muscles having a width of about 300 msec and had a diastolic slow depolarization (pacemaker potential).

### Example 8

### (1) Induction of human cord blood mesenchymal stem cells into cardiac muscles

Studies on the capability of human cord blood mesenchymal stem cells to be differentiated into cardiac muscles cells were made as follows.
Human cord blood mesenchymal stem cells described in a document [Molecular Biology of the Cell., 16, 1491-1499 (05)] were infected with an adenovirus which expresses GFP according to the method described in a document [J. Gene Med., 6, 833-845 (04)]. Specifically, the human cord blood mesenchymal stem cells were inoculated at a density of 5 × 10⁴/cm² in a DMEM medium containing 10% FCS in a 6-cm culture dish, and, on the next day, were infected with an adenovirus which expresses GFP. The supernatant was removed followed by further culture in the same medium in an incubator under conditions of 37°C and CO₂ concentration 5%. The expression of GFP was confirmed with a fluorescent confocal microscope.

The coculture with mouse fetus-derived cardiac muscle cells was carried out under conditions described in a document [J. Gene Med., 6, 833-845 (04)]. Specifically, 5-aza-C untreated human cord blood mesenchymal stem cells were peeled off from the culture dish by PBS (phosphate-buffered saline) containing 0.05% trypsin (manufactured by GIBCO) and 0.25 mmol/l EDTA (ethylenediamine-N,N,N',N'-tetraacetic acid) and were overlaid onto mouse fetus-derived cardiac muscle cells which had been previously inoculated at a density of 5 × 10³/cm². The human cord blood mesenchymal stem cells were induced into cardiac muscle cells by culture in a 10% FCS-containing DMEM medium in an incubator under conditions of CO₂ concentration 5% and 37°C. The human cord blood mesenchymal stem cells not to be cocultured were likewise peeled off from the culture dish by PBS containing 0.05% trypsin and 0.25 mmol/l EDTA and was then cultured in a 10% FCS-containing DMEM medium in an incubator under conditions of CO₂ concentration 5% and 37°C.

### (2) Cardiomyogenic differentiation ratio of human cord blood mesenchymal stem cells

As a result of visual observation, it was found that, for the culture dish containing human cord blood mesenchymal stem cells cocultured with mouse fetus-derived cardiac muscle cells for one week, about 80% of the cells caused automonous beating, and the cells which emit GFP fluorescence were synchronized with each other and were contracted. Since, however, all the cells are synchronized, it was difficult to accurately evaluate the proportion of cells which cause cardiac muscle induction. Accordingly, for the human cord blood mesenchymal stem cells, a sample, which had been cocultured with mouse fetus-derived cardiac muscle cells for one week, and a sample, which had been solely cultured without coculture, were provided. In order to remove the serum in the culture, the sample was washed once with PBS. PBS containing 0.05% trypsin and 0.25 mmol/l EDTA was then added thereto, and the mixture was enzymatically reacted in a humidified incubator of 37°C for 5 min. The cells were peeled off as a lump from the Petri culture dish. The peeled tissue was placed in PBS containing 0.5% collagenase (Typell, manufactured by Worthington) and 10 mmol/l 2,3-butanedione monoxime (hereinafter abbreviated to BDM), and a reaction was further allowed to proceed for additional 20 min in a thermostatic chamber of 37°C, and cells were isolated.

For the cells, staining was carried out using an antibody (Monoclonal mouse anti-cardiac Troponin I for human Catalogue # 4T21, manufactured by HyTest) to Troponin I which is an intracellular antigen and is a human cardiac muscle-specific protein. At the outset, a slide glass on its surface was coated with poly-L-lysine (P8920, manufactured by Sigma). The slide glass was previously washed with 1% HCl-containing 70% ethanol, and poly-L-lysine diluted with pure water by a factor of 10 was added thereto, followed by standing at room temperature for 5 min. Thereafter, poly-L-lysine was removed, and the slide glass was thoroughly dried at 60°C for one hr. A suspension of the human cord blood mesenchymal stem cells were placed on the lysine-coated slide glass for one hr to strongly bond the cell line to the slide glass. Thereafter, the cells were fixed with 4% paraformaldehyde, followed by immersion in PBS containing 0.02% Triton-X for 20 min to destroy cell membranes. A solution prepared by diluting an anti-human cardiac muscle Troponin I antibody as a primary antibody with PBS by a factor of 400 was then added thereto, and a reaction was allowed to proceed with moisture keeping in a refrigerator for one day, followed by washing with PBS ten times to remove the residual antibody. A secondary antibody (Anti Mouse TRITC, manufactured by Sigma) solution was added thereto, and a reaction was allowed to proceed with moisture keeping at room temperature for 30 min. The sample was observed under a confocal laser microscope (FV1000, manufactured by Olympus). As a result, it was found that an image of human cardiac muscle Troponin I which had been stained in a donut form while avoiding the nucleus in its center, was observed. This was determined to be positive, and the proportion of Troponin I expressed cells in the GFP-positive cells was calculated as a cardiomyogenic differentiation ration. As a result, it was found that Troponin-I was expressed in about 45% of human cord blood mesenchymal stem cells.

It was found that, regardless of the 5-aza-C treatment, the proportion of the Troponin I-positive cells was very low for the human cord blood mesenchymal stem cells which had been solely cultured without coculture with the mouse fetus-derived cardiac muscle cells (not more than 1% of the GFP-positive cells). On the other hand, for the case where coculture with mouse fetus-derived cardiac muscle cells was carried out, the proportion of Troponin I-positive cells was about 45% even for the human cord blood mesenchymal stem cells which had not been subjected to 5-aza-C treatment which had hitherto been considered indispensable for cardiac muscle induction. The above results demonstrate that, as compared with the proportion of induction into human cardiac muscle cells in the prior art technique (generally not more than 1%), the mesenchymal stem cells derived from human placenta and umbilical cord, which, when cocultured with cardiac muscle cells, cause autonomous beating at a very high percentage and are already induced into cardiac muscles having a physiological function on the culture dish, regardless of the 5-aza-C treatment.

### (2) Cardiac muscle-specific action potential obtained from human cord blood mesenchymal stem cells

The action potential was measured based on the method described in a document [J. Gene Med., 6, 833-845 (04)] in the same manner as in Example 2. As a result, the recorded action potential was that characteristic of cardiac muscles having a width of about 200 msec, and a diastolic slow depolarization (pacemaker potential) could not be recorded from the cells.

## Claims

1. A cell capable of differentiating into a cardiac muscle cell, which originates in an endometrial tissue or from an endometrial tissue isolated from a menstrual blood, or originates in mesenchymal stem cells isolated from a menstrual blood, a cord blood or an appendage of a fetus.

2. The cell according to claim 1, which is isolated for the use of the regeneration of self or other's cardiac muscle cells.

3. The cell according to claim 1 or 2, which is immortalized.

4. The cell according to claim 3, wherein the cell is immortalized by intracellular expression of telomerase or Bmi-1.

5. The cell according to claim 3, which is immortalized by intracellular expression of an E6 or E7 gene of a human papilloma virus.

6. The cell according to any one of claims 1 to 5, which is able to differentiate into a ventricular cardiac muscle cell or sinus node cell.

7. The cell according to any one of claims 1 to 6, which is able to differentiate into a cardiac muscle cell by coculture with a cardiac muscle-derived cell.

8. The cell according to claim 7, wherein the cardiac muscle-derived cell is a mammalian cell.

9. The cell according to claim 8, wherein the origin of mammalian cell is selected from the group consisting of mice, rats, guinea pigs, hamsters, rabbits, cats, dogs, sheep, pigs, cattle, goats, monkeys, and humans.

10. The cell according to claim 5, which is CD140a-negative.

11. The cell according to claim 5, which is positive against at least one of CD13, CD29, CD44, CD54, CD55, CD59, CD90, and CD166.

12. The cell according to claim 5, which is negative against at least one of CD14, CD24, CD31, CD34, CD45, CD50, and Flk-1.

13. The cell according to any one of claims 1 to 12, which is an hEPC100 cell (FERM AP-20368) or an hEPC214 cell (FERM AP-20369) cell.

14. The cell according to claim 1, wherein the appendage of the fetus is placenta, umbilical cord, or amnion.

15. A cardiac muscle cell or its precursor cell which is differentiated from a cell according to any one of claims 1 to 14.

16. A method for preparing a cardiac muscle cell, comprising:
obtaining an endometrial cell from an endometrial tissue or a menstrual blood, or obtaining a mesenchymal stem cell isolated from a menstrual blood, a cord blood or an appendage of a fetus; and
inducing the differentiation of the cell into a cardiac muscle cell.

17. The method according to claim 16, the differentiation is carried out by at least one induction selected from the group consisting of coculture with a cardiac muscle cell, differentiation by demethylation of DNA, differentiation by a factor which develops in a fetal cardiogenesis region, or by a factor which acts on differentiation into a cardiac muscle cell at a fetal cardiogenesis stage, and differentiation by a culture supernatant of a cell capable of differentiating into a cardiac muscle cell, or a culture supernatant of a cardiac muscle cell differentiated from the cell.

18. The method according to claim 17, wherein the differentiation is carried out by coculture with a cardiac muscle cell.

19. The method according to claim 18, wherein the differentiation is carried out by a combination of coculture with a cardiac muscle cell with at least one induction selected from the group consisting of, differentiation by demethylation of DNA, differentiation by a factor which develops in a fetal cardiogenesis region, or by a factor which acts on differentiation into a cardiac muscle cell at a fetal cardiogenesis stage, and differentiation by a culture supernatant of a cell capable of differentiating into a cardiac muscle cell, or a culture supernatant of a cardiac muscle cell differentiated from the cell.

20. The method according to claim 17 or 19, wherein the demethylation of DNA is carried out by at least one material selected from the group consisting of demethylase, 5-azacytidine, and dimethyl sulfoxide (DMSO).

21. The method according to any one of claims 16 to 20, wherein the endometrical cell is immortalized before differentiation into a cardiac muscle cell.

22. The method according to claim 21, wherein the cell is immortalized by intracellular expression of telomerase or Bmi-1.

23. The method according to claim 21, wherein the cell is immortalized by intracellular expression of an E6 or E7 gene of a human papilloma virus.

24. The method according to any one of claims 17 to 23, wherein the cardiac muscle-derived cell to be cocultured originates in a mammal.

25. The method according to claim 24, wherein the mammal is selected from the group consisting of mice, rats, guinea pigs, hamsters, rabbits, cats, dogs, sheep, pigs, cattle, goats, monkeys, and humans.

26. A medicament comprising as an active ingredient a cell according to any one of claims 1 to 14.

27. The medicament according to claim 26 for use as a cardiac anagenetic agent.

28. The medicament according to claim 26 for use as a therapeutic agent for cardiac diseases.

29. The medicament according to claim 26 for use in the treatment of cardiac infarction, ischemic heart diseases, congestive cardiac failure, arrhythmia, hypertrophic cardiomyopathy, dilated cardiomyopathy, myocarditis, or valvular disease.

30. A thrapeutic agent for congenital heart diseases, comprising as an active ingredient a cell according to any one of claims 1 to 15 into which a wild type gene relative to a mutant gene for congenital cardiac gene diseases has been introduced.

31. A process for preparing a cell capable of differentiating into a cardiac muscle cell, comprising introducing a normal gene into cells capable of differentiating into a cardiac muscle cell isolated from a patient suffering from a congenital cardiac gene disease, and then culturing the cell for transplantation into the patient.

32. An antibody capable of recognizing an antigen which develops in a cell according to any one of claims 1 to 15.

33. A method for isolating a cell specifically according to any one of claims 1 to 15 using an antibody according to claim 32.

34. A method for obtaining a surface antigen specific for a cell according to any one of claims 1 to 15, **characterized by** using a cell according to any one of claims 1 to 15.

35. A method for screening a factor for growing a cell according to any one of claims 1 to 15, **characterized by** using a cell according to any one of claims 1 to 15.

36. A method for screening a factor for inducing the differentiation of a cell according to any one of claims 1 to 15 into a cardiac muscle cell, **characterized by** using a cell according to any one of claims 1 to 15.

37. A culture supernatant containing a cell according to any one of claims 1 to 13.

38. A method for differentiation inducting a cell according to any one of claims 1 to 13 into a cardiac muscle cell, **characterized by** using a culture supernatant according to claim 37.
